# EUROPEAN PATENT SPECIFICATION

(11) **EP 1 095 150 B1**
(45) Date of publication and mention of the grant of the patent: **15.02.2006**
(21) Application number: 99933660.5
(22) Date of filing: 02.07.1999
(51) Int. Cl.: C12N 15/53, C12N 9/02, C12Q 1/26, C12N 15/81, C12N 1/19

(54) **FORMALDEHYDE DEHYDROGENASE GENES FROM METHYLOTROPHIC YEASTS**
FORMALDEHYDE DEHYDROGENASE GENE AUS METHYLOTROPHEN HEFEN
GENES DE FORMALDEHYDE DESHYDROGENASE A PARTIR DE LEVURES METHYLOTROPHES

(30) Priority: 03.07.1998 US 91699 P
(43) Date of publication of application: 02.05.2001
(73) Proprietor: RESEARCH CORPORATION TECHNOLOGIES, INC., Tucson, Arizona 85711-3335 (US)
(72) Inventor: CREGG, James, M., Portland, OR 97229 (US)
(74) Representative: HOFFMANN EITLE
(86) International application number: PCT/US1999/015016
(87) International publication number: WO 2000/001829

(56) References cited:
- S. SHEN ET AL.: "A strong nitrogen source-regulated promoter for controlled expression of foreign genes in the yeast Pichia pastoris." GENE, vol. 216, no. 1, 17 August 1998 (1998-08-17), pages 93-102, XP002125604 Amsterdam
- M.A. JOHNSON ET AL.: "Positive selection of novel peroxisome biogenesis-defective mutants of the yeast pichia pastoris" GENETICS, vol. 151, no. 4, April 1999 (1999-04), pages 1379-1391, XP002125605
- K SASNAUSKAS ET AL: "Cloning and analysis of a Candida maltosa gene which confers resistance to formaldehyde in Saccharomyces cerevisiae" GENE,NL,ELSEVIER BIOMEDICAL PRESS. AMSTERDAM, vol. 122, no. 122, page 207-211-211 XP002100683 ISSN: 0378-1119
- E.P. WEHNER ET AL.: "Molecular structure and genetic regulation of SFA, a gene responsible for resistance to formaldehyde in Saccharomyces cerevisiae, and characterization of its protein product." MOLECULAR AND GENERAL GENETICS, vol. 237, 1993, pages 351-358, XP000857482
- M-W HUR ET AL.: "Cloning and characterization of the ADH5 gene encoding human alcohol dehydrogenase 5, formaldehyde dehydrogenase." GENE, vol. 121, 1992, pages 305-311, XP000857486
- P. RATHNA GIRI ET AL.: "Cloning and comparative mapping of a human class III (X) alcohol dehydrogenase cDNA" BIOCHEMICAL AND BIOPHYSICAL RESEARCH COMMUNICATIONS, vol. 164, no. 1, 16 October 1989 (1989-10-16), pages 453-460, XP000857485
- M.A. JOHNSON ET AL.: "Positive selection for peroxisome-deficient mutants of the methylotrophic yeast Pichia pastoris." MOLECULAR BIOLOGY OF THE CELL, ANNUAL MEETING OF THE 6TH INTERNATIONAL CONGRESS ON CELL BIOLOGY AND THE 36TH AMERICAN SOCIETY FOR CELL BIOLOGY, vol. 7, no. SUPPL, 7 - 11 December 1996, page 497A XP002125606 SAN FRANSCISCO, CALIFORNIA, USA.
- M.A. VAN DEN BERG: "Expression cassettes for formaldehyde and fluoroacetate resistance, two dominant markers in Saccharomyces cerevisiae." YEAST, vol. 13, 1997, pages 551-559, XP002125607
- J.F. TSCHOPP ET AL.: "Expression of the lacZ gene from two methanol-regulated promoters in Pichia pastoris." NUCLEIC ACIDS RESEARCH, vol. 15, no. 9, 1987, pages 3859-3876, XP002125608 cited in the application
- M. GREY ET AL.: "Overexpression of ADH1 confers hyper-resistance to formaldehyde in Saccharomyces cerevisiae" CURRENT GENETICS, vol. 29, 1996, pages 437-440, XP002125609
- O.V. STASYK ET AL.: "Mutants of the methylotrophic yeast Hansenula polymorpha with impaired catabolite repression." MICROBIOLOGY, vol. 66, no. 6, 1997, pages 755-760, XP002125611
- M.R. FERNANDEZ: "Formaldehyde dehydrogenase from yeast and plant." ADVANCES IN EXPERIMENTAL MEDICINE AND BIOLOGY, vol. 414, 1997, pages 373-381, XP002125610
- L. UOTILA: "Glutathione-dependent oxidoreductases: formaldehyde dehydrogenase" COENZYMES COFACTORS, vol. 3, 1989, pages 517-51, XP002125612

### SEQUENCE LISTING

<110> Cregg, James M.
<120> FORMALDEHYDE DEHYDROGENASES GENES FROM METHYLOTROPHIC YEASTS
<130> RESEARCH CORPORATION
<140> Unassigned
   <141> 1999-07-02
<160> 5
<170> PatentIn Ver. 2.0
<210> 1
   <211> 2774
   <212> DNA
   <213> Pichia pastoris
<220>
   <221> CDS
   <222> (598)..(615)
<220>
   <221> CDS
   <222> (730)..(1851)
<400> 1
   gcatgcagga atctctggca cggtgctaat ggtagttatc caacggagct gaggtagtcg 60
   atatatctgg atatgccgcc tataggataa aaacaggaga gggtgaacct tgcttatggc 120
   tactagattg ttcttgtact ctgaattctc attatgggaa actaaactaa tctcatctgt 180
   gtgttgcagt actattgaat cgttgtagta tctacctgga gggcattcca tbaattagtg 240
   agataacaga gttgggtaac tagagagaat aatagacgta tgcatgatta ctacacaacg 300
   gatgtcgcac tctttcctta gttaaaacta tcatccaatc acaagatgcg ggctggaaag 360
   acttgctccc gaaggataat cttctgcttc tatctccctt cctcatatgg tttcgcaggg 420
   ctcatgcccc ttcttccttc gaactgcccg atgaggaagt ccttagccta tcaaagaatt 480
   cgggaccatc atcgattttt agagccttac ctgatcgcaa tcaggatttc actactcata 540
   taaatacatc gctcaaagct ccaactttgc ttgttcatac aattcttgat attcaca 597
   atg tct acc gaa ggt caa gtaagttcaa tcaaagtaat tgtttgggag         645
   Met Ser Thr Glu Gly Gln
     1               5
   ggaagaagat tgttttattg cgaacctttc aatatcttac ccgactaaat aaccattaca 705
   gtgaattttt tactaactat atag atc atc aaa tgt aag gca gct gtt gcc   756
                               Ile Ile Lys Cys Lys Ala Ala Val Ala
                                            10                  15
   tgg gag gca gga aag gat ctc tct att gag gag att gag gtt ctt cct   804
   Trp Glu Ala Gly Lys Asp Leu Ser Ile Glu Glu Ile Glu Val Leu Pro
                   20                  25                  30
   cca aga gcc cat gaa gtt aga gtg aaa gtg gaa ttc act ggt gta tgc   852
   Pro Arg Ala His Glu Val Arg Val Lys Val Glu Phe Thr Gly Val Cys
               35                  40                  45
   cac act gat gct tac acg ctt tct ggt gca gat gca gag gga agt ttc   900
   His Thr Asp Ala Tyr Thr Leu Ser Gly Ala Asp Ala Glu Gly Ser Phe
           50                  55                  60
   cct gtt gtg ttc ggc cat gaa ggt gct ggt gtt gtc gag tca gtt gga   948
   Pro Val Val Phe Gly His Glu Gly Ala Gly Val Val Glu Ser Val Gly
       65                  70                  75
   gaa ggt gtt gag tcc gtg aag gtt ggg gat tct gta gtg ctt ctg tac   996
   Glu Gly Val Glu Ser Val Lys Val Gly Asp Ser Val Val Leu Leu Tyr
    80                  85                  90                  95
   act cct gag tgc aga gag tgc aag ttc tgt ctg tct ggt aag acg aac   1044
   Thr Pro Glu Cys Arg Glu Cys Lys Phe Cys Leu Ser Gly Lys Thr Asn
                   100                 105                 110
   ctc tgt ggt aaa atc aga gcc acc cag ggt aaa ggt ttg tta cca gac   1092
   Leu Cys Gly Lys Ile Arg Ala Thr Gln Gly Lys Gly Leu Leu Pro Asp
               115                 120                 125
   ggg act tct cgt ttc cgt tgt aag ggc aag gat ttg ttt cac tat atg   1140
   Gly Thr Ser Arg Phe Arg Cys Lys Gly Lys Asp Leu Phe His Tyr Met
           130                 135                 140
   gga tgt tct tcc ttt tct caa tac act gtg gtg gct gac atc tca gtg   1188
   Gly Cys Ser Ser Phe Ser Gln Tyr Thr Val Val Ala Asp Ile Ser Val
       145                 150                 155
   gtt aaa gtc caa gac gaa gct cct aag gac aag aca tgt ctg ttg ggt   1236
   Val Lys Val Gln Asp Glu Ala Pro Lys Asp Lys Thr Cys Leu Leu Gly
   160                 165                 170                 175
   tgt ggt gtt acc aca ggg tac ggt gct gct atc aac act gct aag atc   1284
   Cys Gly Val Thr Thr Gly Tyr Gly Ala Ala Ile Asn Thr Ala Lys Ile
                   180                 185                 190
   tct aag ggt gac aag atc ggt gtg ttt ggt gct gga tgt att gga tta   1332
   Ser Lys Gly Asp Lys Ile Gly Val Phe Gly Ala Gly Cys Ile Gly Leu
               195                 200                 205
   tct gtc atc caa ggt gca gtt tcc aaa ggt gca agc gag att att gta   1380
   Ser Val Ile Gln Gly Ala Val Ser Lys Gly Ala Ser Glu Ile Ile Val
           210                 215                 220
   att gac atc aat gat tca aag aag gca tgg gcg gac caa ttt ggt gca   1428
   Ile Asp Ile Asn Asp Ser Lys Lys Ala Trp Ala Asp Gln Phe Gly Ala
       225                 230                 235
   act aag ttt gtc aat cct aca acc tta cca gaa ggt acc aat att gtt   1476
   Thr Lys Phe Val Asn Pro Thr Thr Leu Pro Glu Gly Thr Asn Ile Val
   240                 245                 250                 255
   gac tac ttg att gat atc act gac gga ggc ttt gac tat acc ttc gac   1524
   Asp Tyr Leu Ile Asp Ile Thr Asp Gly Gly Phe Asp Tyr Thr Phe Asp
                   260                 265                 270
   tgt acc ggt aat gtt caa gta atg aga aat gca ctt gaa tct tgc cac   1572
   Cys Thr Gly Asn Val Gln Val Met Arg Asn Ala Leu Glu Ser Cys His
               275                 280                 285
   aag ggt tgg ggt gag tcg atc atc atc ggt gtc gct gct gct ggt aaa   1620
   Lys Gly Trp Gly Glu Ser Ile Ile Ile Gly Val Ala Ala Ala Gly Lys
           290                 295                 300
   gaa atc tct acc cgt cct ttc cag ttg gtt act ggc aga gtc tgg aga   1668
   Glu Ile Ser Thr Arg Pro Phe Gln Leu Val Thr Gly Arg Val Trp Arg
       305                 310                 315
   gga tgc gcc ttt gga ggt atc aag gga cgt act caa atg cca tct ttg   1716
   Gly Cys Ala Phe Gly Gly Ile Lys Gly Arg Thr Gln Met Pro Ser Leu
   320                 325                 330                 335
   gtt cag gac tat ctt gat ggt aag att aaa gtt gac gag ttt atc aca   1764
   Val Gln Asp Tyr Leu Asp Gly Lys Ile Lys Val Asp Glu Phe Ile Thr
                   340                 345                 350
   cac aga cat gac ctg gac aac atc aac aaa gca ttt cat gac atg cat    1812
   His Arg His Asp Leu Asp Asn Ile Asn Lys Ala Phe His Asp Met His
               355                 360                     365
   gct gga aac tgt att cgt gct gtg att act atg cac taa gtacgacgta    1861
   Ala Gly Asn Cys Ile Arg Ala Val Ile Thr Met His
           370                 375                 380
   tgatgaatga atgagttatg taaggcccga tctcagctag gacgtttata gacctatgta 1921
   tatatatgta tgtatacgta tatacctcaa actcatttta tggctatagg aaggattgtt 1981
   ttcatcgtta tgtccgaaga tacatcaata cagcgtttct tgatttgtac caaacactcc 2041
   ccaggtagat tctccagtct cgctagttac cttcgcggta ttgatcgacg ctctgtgaaa 2101
   aaataaaaaa aaaatgtcaa tagtatcgag gttggcttca aaatcaccag cgttctgtat 2161
   cgggttccgt ttttgagctt ctactttcct tatatccatc atacattcct gtgcttgaat 2221
   tcagcagtag aatgtccgat cgatatattg tgttgcatat caacaccacc gccaatgaga 2281
   gttctcagca gttcaaacgc gacccttcag agattattga gctagcatgg gttctgctag 2341
   atcctggaac caactttgaa atagttggta ggggaagtgt tctggctaaa ccattcaaca 2401
   ctcctataac cccgctttgt accagcatga caactcttac atgggaaagt gtcaagaacg 2461
   ctggttcact caaggatgcg ctagaggagc ttagcagatt tattgactca aacttggtca 2521
   gcaacggctt gtcattcagt tttataactc tgaatgcctg ggatctccgc ttgaaattac 2581
   ccaaggagtc gcgtgaaaga agtatagccc ttcccgcgta cttggactta cccaagtact 2641
   ttgatcttag aaaagaattc tgtagatggg cccaaaaatc atctgcattg actacaaacg 2701
   gtaatcacat gagtttagct tatatggtat ctaaacttga aacagaagct agtttggttt 2761
   tggacgagga tcc                                                    2774
<210> 2
   <211> 379
   <212> PRT
   <213> Pichia pastoris
<400> 2
   Met Ser Thr Glu Gly Gln Ile Ile Lys Cys Lys Ala Ala Val Ala Trp
   1 5 10 15
   Glu Ala Gly Lys Asp Leu Ser Ile Glu Glu Ile Glu Val Leu Pro Pro
   20 25 30
   Arg Ala His Glu Val Arg Val Lys Val Glu Phe Thr Gly Val Cys His
   35 40 45
   Thr Asp Ala Tyr Thr Leu Ser Gly Ala Asp Ala Glu Gly Ser Phe Pro
   50 55 60
   Val Val Phe Gly His Glu Gly Ala Gly Val Val Glu Ser Val Gly Glu
   65 70 75 80
   Gly Val Glu Ser Val Lys Val Gly Asp Ser Val Val Leu Leu Tyr Thr
   85 90 95
   Pro Glu Cys Arg Glu Cys Lys Phe Cys Leu Ser Gly Lys Thr Asn Leu
   100 105 110
   Cys Gly Lys Ile Arg Ala Thr Gln Gly Lys Gly Leu Leu Pro Asp Gly
   115 120 125
   Thr Ser Arg Phe Arg Cys Lys Gly Lys Asp Leu Phe His Tyr Met Gly
   130 135 140
   Cys Ser Ser Phe Ser Gln Tyr Thr Val Val Ala Asp Ile Ser Val Val
   145 150 155 160
   Lys Val Gln Asp Glu Ala Pro Lys Asp Lys Thr Cys Leu Leu Gly Cys
   165 170 175
   Gly Val Thr Thr Gly Tyr Gly Ala Ala Ile Asn Thr Ala Lys Ile Ser
   180 185 190
   Lys Gly Asp Lys Ile Gly Val Phe Gly Ala Gly Cys Ile Gly Leu Ser
   195 200 205
   Val Ile Gln Gly Ala Val Ser Lys Gly Ala Ser Glu Ile Ile Val Ile
   210 215 220
   Asp Ile Asn Asp Ser Lys Lys Ala Trp Ala Asp Gln Phe Gly Ala Thr
   225 230 235 240
   Lys Phe Val Asn Pro Thr Thr Leu Pro Glu Gly Thr Asn Ile Val Asp
   245 250 255
   Tyr Leu Ile Asp Ile Thr Asp Gly Gly Phe Asp Tyr Thr Phe Asp Cys
   260 265 270
   Thr Gly Asn Val Gln Val Met Arg Asn Ala Leu Glu Ser Cys His Lys
           275                 280                 285
   Gly Trp Gly Glu Ser Ile Ile Ile Gly Val Ala Ala Ala Gly Lys Glu
       290                 295                 300
   Ile Ser Thr Arg Pro Phe Gln Leu Val Thr Gly Arg Val Trp Arg Gly
   305                 310                 315                 320
   Cys Ala Phe Gly Gly Ile Lys Gly Arg Thr Gln Met Pro Ser Leu Val
                   325                 330                 335
   Gln Asp Tyr Leu Asp Gly Lys Ile Lys Val Asp Glu Phe Ile Thr His
               340                 345                 350
   Arg His Asp Leu Asp Asn Ile Asn Lys Ala Phe His Asp Met His Ala
           355                 360                 365
   Gly Asn Cys Ile Arg Ala Val Ile Thr Met His
       370                 375
<210> 3
   <211> 597
   <212> DNA
   <213> Pichia pastoris
<400> 3
   gcatgcagga atctctggca cggtgctaat ggtagttatc caacggagct gaggtagtcg 60
   atatatctgg atatgccgcc tataggataa aaacaggaga gggtgaacct tgcttatggc 120
   tactagattg ttcttgtact ctgaattctc attatgggaa actaaactaa tctcatctgt 180
   gtgttgcagt actattgaat cgttgtagta tctacctgga gggcattcca tgaattagtg 240
   agataacaga gttgggtaac tagagagaat aatagacgta tgcatgatta ctacacaacg 300
   gatgtcgcac tctttcctta gttaaaacta tcatccaatc acaagatgcg ggctggaaag 360
   acttgctccc gaaggataat cttctgcttc tatctccctt cctcatatgg tttcgcaggg 420
   ctcatgcccc ttcttccttc gaactgcccg atgaggaagt ccttagccta tcaaagaatt 480
   cgggaccatc atcgattttt agagccttac ctgatcgcaa tcaggatttc actactcata 540
   taaatacatc gctcaaagct ccaactttgc ttgttcatac aattcttgat attcaca    597
<210> 4
   <211> 300
   <212> DNA
   <213> Pichia pastoris
<400> 4
   gtacgacgta tgatgaatga atgagttatg taaggcccga tctcagctag gacgtttata 60
   gacctatgta tatatatgta tgtatacgta tatacctcaa actcatttta tggctatagg 120
   aaggattgtt ttcatcgtta tgtccgaaga tacatcaata cagcgtttct tgatttgtac 180
   caaacactcc ccaggtagat tctccagtct cgctagttac cttcgcggta ttgatcgacg 240
   ctctgtgaaa aaataaaaaa aaaatgtcaa tagtatcgag gttggcttca aaatcaccag 300
<210> 5
   <211> 1293
   <212> DNA
   <213> Pichia pastoris
<220>
   <221> CDS
   <222> (1)..(18)
   <223> genomic, double stranded
<220>
   <221> CDS
   <222> (133)..(1293)
   <223> genomic, double stranded
<400> 5
   atg tct acc gaa ggt caa gtaagttcaa tcaaagtaat tgtttgggag          48
   Met Ser Thr Glu Gly Gln
     1               5
   ggaagaagat tgttttattg cgaacctttc aatatcttac ccgactaaat aaccattaca 108
   gtgaattttt tactaactat atag atc atc aaa tgt aag gca gct gtt gcc    159
                               Ile Ile Lys Cys Lys Ala Ala Val Ala
                                           10                  15
   tgg gag gca gga aag gat ctc tct att gag gag att gag gtt ctt cct   207
   Trp Glu Ala Gly Lys Asp Leu Ser Ile Glu Glu Ile Glu Val Leu Pro
                   20                  25                  30
   cca aga gcc cat gaa gtt aga gtg aaa gtg gaa ttc act ggt gta tgc   255
   Pro Arg Ala His Glu Val Arg Val Lys Val Glu Phe Thr Gly Val Cys
               35                  40                  45
   cac act gat gct tac acg ctt tct ggt gca gat gca gag gga agt ttc      303
   His Thr Asp Ala Tyr Thr Leu Ser Gly Ala Asp Ala Glu Gly Ser Phe
           50                  55                  60
   cct gtt gtg ttc ggc cat gaa ggt gct ggt gtt gtc gag tca gtt gga      351
   Pro Val Val Phe Gly His Glu Gly Ala Gly Val Val Glu Ser Val Gly
       65                  70                  75
   gaa ggt gtt gag tcc gtg aag gtt ggg gat tct gta gtg ctt ctg tac      399
   Glu Gly Val Glu Ser Val Lys Val Gly Asp Ser Val Val Leu Leu Tyr
    80                  85                  90                  95
   act cct gag tgc aga gag tgc aag ttc tgt ctg tct ggt aag acg aac      447
   Thr Pro Glu Cys Arg Glu Cys Lys Phe Cys Leu Ser Gly Lys Thr Asn
                   100                 105                 110
   ctc tgt ggt aaa atc aga gcc acc cag ggt aaa ggt ttg tta cca gac      495
   Leu Cys Gly Lys Ile Arg Ala Thr Gln Gly Lys Gly Leu Leu Pro Asp
               115                 120                 125
   ggg act tct cgt ttc cgt tgt aag ggc aag gat ttg ttt cac tat atg      543
   Gly Thr Ser Arg Phe Arg Cys Lys Gly Lys Asp Leu Phe His Tyr Met
           130                 135                 140
   gga tgt tct tcc ttt tct caa tac act gtg gtg gct gac atc tca gtg      591
   Gly Cys Ser Ser Phe Ser Gln Tyr Thr Val Val Ala Asp Ile Ser Val
       145                 150                 155
   gtt aaa gtc caa gac gaa gct cct aag gac aag aca tgt ctg ttg ggt      639
   Val Lys Val Gln Asp Glu Ala Pro Lys Asp Lys Thr Cys Leu Leu Gly
   160                 165                 170                 175
   tgt ggt gtt acc aca ggg tac ggt gct gct atc aac act gct aag atc      687
   Cys Gly Val Thr Thr Gly Tyr Gly Ala Ala Ile Asn Thr Ala Lys Ile
                   180                 185                 190
   tct aag ggt gac aag atc ggt gtg ttt ggt gct gga tgt att gga tta      735
   Ser Lys Gly Asp Lys Ile Gly Val Phe Gly Ala Gly Cys Ile Gly Leu
               195                 200                 205
   tct gtc atc caa ggt gca gtt tcc aaa ggt gca agc gag att att gta      783
   Ser Val Ile Gln Gly Ala Val Ser Lys Gly Ala Ser Glu Ile Ile Val
           210                 215                 220
   att gac atc aat gat tca aag aag gca tgg gcg gac caa ttt ggt gca   831
   Ile Asp Ile Asn Asp Ser Lys Lys Ala Trp Ala Asp Gln Phe Gly Ala
       225                 230                 235
   act aag ttt gtc aat cct aca acc tta cca gaa ggt acc aat att gtt   879
   Thr Lys Phe Val Asn Pro Thr Thr Leu Pro Glu Gly Thr Asn Ile Val
   240                 245                 250                 255
   gac tac ttg att gat atc act gac gga ggc ttt gac tat acc ttc gac   927
   Asp Tyr Leu Ile Asp Ile Thr Asp Gly Gly Phe Asp Tyr Thr Phe Asp
                   260                 265                 270
   tgt acc ggt aat gtt caa gta atg aga aat gca ctt gaa tct tgc cac   975
   Cys Thr Gly Asn Val Gln Val Met Arg Asn Ala Leu Glu Ser Cys His
               275                 280                 285
   aag ggt tgg ggt gag tcg atc atc atc ggt gtc gct gct gct ggt aaa   1023
   Lys Gly Trp Gly Glu Ser Ile Ile Ile Gly Val Ala Ala Ala Gly Lys
           290                 295                 300
   gaa atc tct acc cgt cct ttc cag ttg gtt act ggc aga gtc tgg aga   1071
   Glu Ile Ser Thr Arg Pro Phe Gln Leu Val Thr Gly Arg Val Trp Arg
       305                 310                 315
   gga tgc gcc ttt gga ggt atc aag gga cgt act caa atg cca tct ttg   1119
   Gly Cys Ala Phe Gly Gly Ile Lys Gly Arg Thr Gln Met Pro Ser Leu
   320                 325                 330                 335
   gtt cag gac tat ctt gat ggt aag att aaa gtt gac gag ttt atc aca   1167
   Val Gln Asp Tyr Leu Asp Gly Lys Ile Lys Val Asp Glu Phe Ile Thr
                   340                 345                 350
   cac aga cat gac ctg gac aac atc aac aaa gca ttt cat gac atg cat   1215
   His Arg His Asp Leu Asp Asn Ile Asn Lys Ala Phe His Asp Met His
               355                 360                 365
   cac aga cat gac ctg gac aac atc aac aaa gca ttt cat gct gga aac   1263
   His Arg His Asp Leu Asp Asn Ile Asn Lys Ala Phe His Ala Gly Asn
           370                 375                 380
   tgt att cgt gct gtg att act atg cac taa                           1293
   Cys Ile Arg Ala Val Ile Thr Met His
       385                 390
<210> 5
   <211> 392
   <212> PRT
   <213> Pichia pastoris
<400> 5
   Met Ser Thr Glu Gly Gln Ile Ile Lys Cys Lys Ala Ala Val Ala Trp
   1               5                   10                  15
   Glu Ala Gly Lys Asp Leu Ser Ile Glu Glu Ile Glu Val Leu Pro Pro
               20                  25                  30
   Arg Ala His Glu Val Arg Val Lys Val Glu Phe Thr Gly Val Cys His
           35                  40                  45
   Thr Asp Ala Tyr Thr Leu Ser Gly Ala Asp Ala Glu Gly Ser Phe Pro
       50                  55                  60
   Val Val Phe Gly His Glu Gly Ala Gly Val Val Glu Ser Val Gly Glu
   65                  70                  75                  80
   Gly Val Glu Ser Val Lys Val Gly Asp Ser Val Val Leu Leu Tyr Thr
                   85                  90                  95
   Pro Glu Cys Arg Glu Cys Lys Phe Cys Leu Ser Gly Lys Thr Asn Leu
               100                 105                 110
   Cys Gly Lys Ile Arg Ala Thr Gln Gly Lys Gly Leu Leu Pro Asp Gly
           115                 120                 125
   Thr Ser Arg Phe Arg Cys Lys Gly Lys Asp Leu Phe His Tyr Met Gly
       130                 135                 140
   Cys Ser Ser Phe Ser Gln Tyr Thr Val Val Ala Asp Ile Ser Val Val
   145                 150                 155                 160
   Lys Val Gln Asp Glu Ala Pro Lys Asp Lys Thr Cys Leu Leu Gly Cys
                   165                 170                 175
   Gly Val Thr Thr Gly Tyr Gly Ala Ala Ile Asn Thr Ala Lys Ile Ser
               180                 185                 190
   Lys Gly Asp Lys Ile Gly Val Phe Gly Ala Gly Cys Ile Gly Leu Ser
           195                 200                 205
   Val Ile Gln Gly Ala Val Ser Lys Gly Ala Ser Glu Ile Ile Val Ile
       210                 215                 220
   Asp Ile Asn Asp Ser Lys Lys Ala Trp Ala Asp Gln Phe Gly Ala Thr
   225                 230                 235                 240
   Lys Phe Val Asn Pro Thr Thr Leu Pro Glu Gly Thr Asn Ile Val Asp
                   245                 250                 255
   Tyr Leu Ile Asp Ile Thr Asp Gly Gly Phe Asp Tyr Thr Phe Asp Cys
               260                 265                 270
   Thr Gly Asn Val Gln Val Met Arg Asn Ala Leu Glu Ser Cys His Lys
           275                 280                 285
   Gly Trp Gly Glu Ser Ile Ile Ile Gly Val Ala Ala Ala Gly Lys Glu
       290                 295                 300
   Ile Ser Thr Arg Pro Phe Gln Leu Val Thr Gly Arg Val Trp Arg Gly
   305                 310                 315                 320
   Cys Ala Phe Gly Gly Ile Lys Gly Arg Thr Gln Met Pro Ser Leu Val
                   325                 330                 335
   Gln Asp Tyr Leu Asp Gly Lys Ile Lys Val Asp Glu Phe Ile Thr His
               340                 345                 350
   Arg His Asp Leu Asp Asn Ile Asn Lys Ala Phe His Asp Met His His
           355                 360                 365
   Arg His Asp Leu Asp Asn Ile Asn Lys Ala Phe His Ala Gly Asn Cys
       370                 375                 380
   Ile Arg Ala Val Ile Thr Met His
   385                 390

## Description

### BACKGROUND OF THE INVENTION

*Pichia* is a methylotrophic yeast that is widely used for the production of heterologous proteins of industrial and academic interest (Cregg, 1998; Higgins and Cregg, 1998). FLD is an important enzyme in the utilization of methanol as a carbon and energy source (Veenhuis et al., 1983). In methylotrophic yeasts, the methanol metabolic pathway is thought to be nearly the same, beginning with the oxidation of methanol to formaldehyde by alcohol oxidase (AOX), a hydrogen peroxide-producing oxidase that is sequestered in an organelle called the peroxisome. Hydrogen peroxide is then degraded to oxygen and water by catalase, the classic peroxisomal marker enzyme. A portion of the resulting formaldehyde condenses with xylulose-5'-monophosphate in a reaction catalyzed by dihydroxyacetone synthase (DAS), the third peroxisomal methanol pathway enzyme. The products of this reaction, glyceraldehyde-3-phosphate (GAP) and dihydroxyacetone, then leave the peroxisome and enter a cyclic pathway that regenerates xylulose-5'-monophosphate and also generates one net molecule of GAP for every three turns of the cycle. GAP is used for biosynthesis of carbon skeletons for cell growth. Another portion of the formaldehyde leaves the peroxisome and is oxidized to formate by formaldehyde dehydrogenase (FLD) and then to carbon dioxide by formate dehydrogenase (FDH). Both of these reactions produce reducing power in the form of NADH. One model of FLD function is that the NADH generated by FLD and FDH serves as the primary source of energy during growth on methanol (Veenhuis et al., 1983). The second model proposes that most energy for methanol growth comes from the oxidation of one or more of the xylulose-5'-monophosphate cycle intermediates by tricarboxcylic acid cycle enzymes, and that the primary role of FLD is to protect the cell from toxic formaldehyde that accumulates with excess methanol in the medium (Sibirny et al., 1990).

In addition to methanol, FLD is also involved in the metabolism of certain methylated amines (e.g. methylamine and choline) as sole nitrogen sources (Zwart et al., 1980). In this pathway, amine groups are first liberated by a peroxisomal amine oxidase, leaving formaldehyde which is further oxidized by FLD and FDH. When growing on methylamine as sole nitrogen source, high levels of FLD are induced even in the presence of excess glucose. Thus, the primary role of FLD in methylamine metabolism appears to be for protecting cells from the toxic effects of formaldehyde and not for generating carbon or energy.

FLD synthesis is regulated independently in response to either methanol as sole carbon source and energy source or to methylamine as sole nitrogen source. Thus, for example, only low levels of FLD are observed in cells growing on glucose- and ammonium ion-containing medium, whereas on either methanol-ammonium ion or glucose-methylamine media, FLD levels are high.

In the *Pichia* system, most foreign genes are expressed under the transcriptional control of the *P*. *pastoris* alcohol oxidase 1 gene promoter (*P*_{*AOX1*}), the regulatory characteristics of which are well suited for this purpose. The promoter is tightly repressed during growth of the yeast on most common carbon sources, such as glucose, glycerol, or ethanol, but is highly induced during growth on methanol (Tschopp et al., 1987; U.S. Patent No. 4,855,231 to Stroman, D.W., et al). For production of foreign proteins, *P*_{*AOX1*}-controlled expression strains are initially grown on a repressing carbon source to generate biomass and then shifted to methanol as the sole carbon and energy source to induce expression of the foreign gene. One advantage of the *P*_{*AOX1*} regulatory system is that *P. pastoris* strains transformed with foreign genes whose expression products are toxic to the cells can be maintained by growing under repressing conditions.

Although many proteins have been successfully produced using *P*_{*AOX1*}, this promoter is not appropriate or convenient in all settings. For example, in shake-flask cultures, methanol rapidly evaporates, and it is inconvenient to monitor methanol concentrations and repeatedly add the compound to the medium. In addition, the storage of large amounts of methanol needed for the growth and induction of *P*_{*AOX1*}-controlled expression strains in large-volume high-density fermentor cultures is a potential fire hazard. There is a need therefore, for an alternative promoter to *P*_{*AOX1*}, which is both transcriptionally efficient and regulatable by a less volatile and flammable inducer. The present invention provides the *P*. *pastoris* and *Hansenula polymorpha* formaldehyde dehydrogenase gene (FLD) promoter having both properties.

In addition, there is a need for a selectable marker which functions in methylotrophic yeasts other than a selectable marker which is an antibiotic resistance gene. At present, only the *Zeo*^{*R*} gene can be used to transform into *P*. *pastoris* strains independent of their genotype. In addition, *Zeo*^{*R*} is the only that gene can be used to directly select for *P*. *pastoris* strains that receive multiple copies of an expression vector (by increasing the concentration of Zeocin in selective medium) . A second gene which confers resistance to the antibiotic G418 (*G418*^{*R*}) can be used to screen for multicopy expression strains of *P*. *pastoris* but its use requires that an auxotrophic/biosynthetic gene selection marker must also be included in vectors to select for transformants. The *FLD* structural gene of the present invention may be used as a selectable marker in methylotrophic yeast cells and does not confer resistance to antibiotics.

### SUMMARY OF THE INVENTION

The present invention is directed to isolated nucleic acid sequences comprising a formaldehyde dehydrogenase gene (*FLD*) from methylotrophic yeasts. The specification describes the isolated nucleic acids comprise sequences which hybridize under low stringency conditions to at least one of the nucleotide sequences set forth in SEQ ID NO:1, SEQ ID NO:5, or a sequence complementary to the sequence set forth in SEQ ID NOs: 1 or 5.

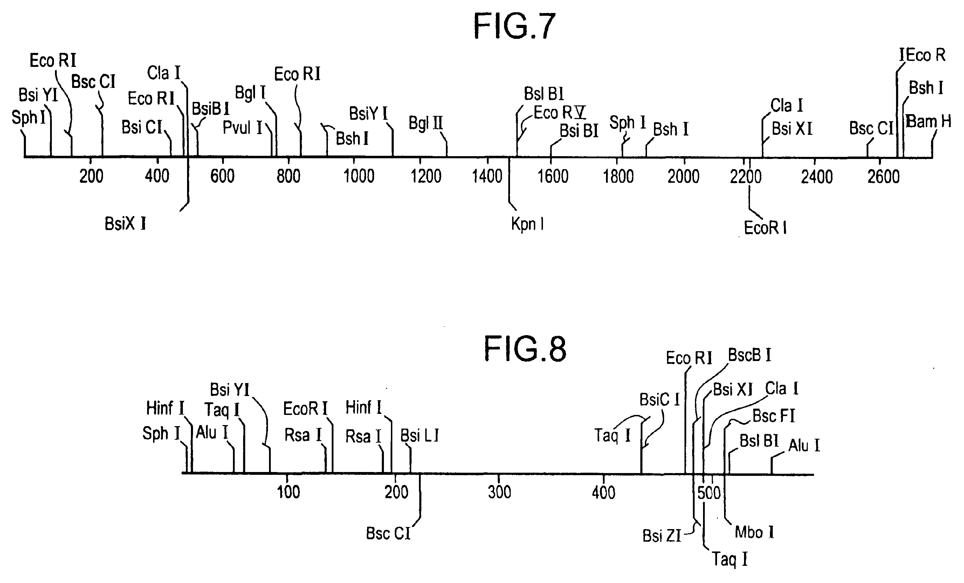

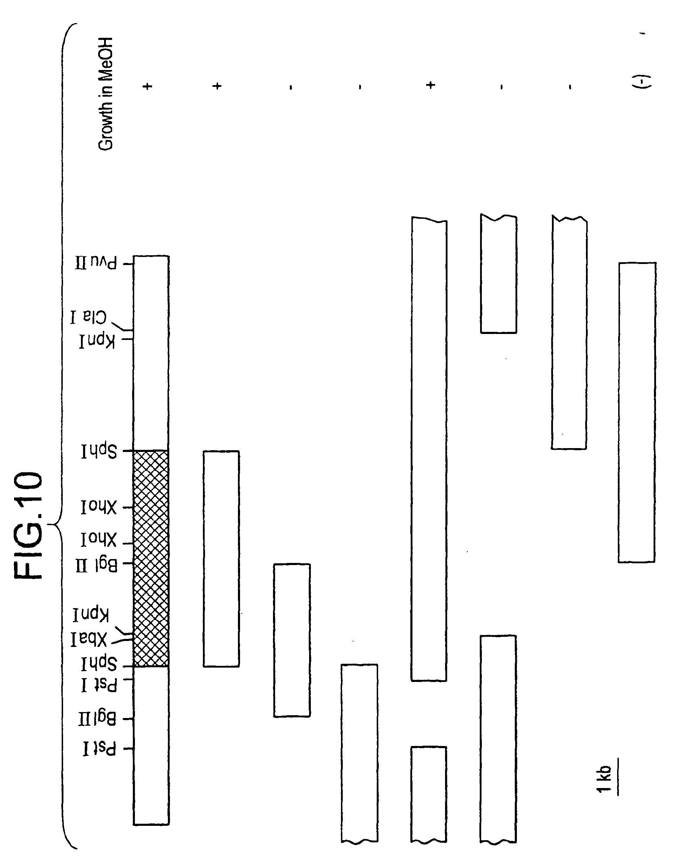

Also provided is an *FLD* gene from *Pichia pastoris* (*FLD1*) having the restriction map set forth in Figure 7 and an *FLD* gene from *Hansenula polymorpha* having the restriction map shown in the cross hatched area of Figure 10.

The specification describes an isolated nucleic acid comprising an *FLD* gene from a methylotrophic yeast with a coding sequence having a sequence homology of about 70% to about 85% when compared to the nucleotide sequence set forth in SEQ ID NO:5. Also described is an isolated nucleic acid comprising an *FLD* gene from a methylotrophic yeast with a coding sequence having a sequence homology of about 85% to about 95% when compared to the nucleotide sequence set forth in SEQ ID NO:5. Also described is an isolated nucleic acid comprising an *FLD* gene from a methylotrophic yeast with a coding sequence having a sequence homology of greater than about 95% when compared to the nucleotide sequence set forth in SEQ ID NO:5. Isolated nucleic acids comprising the sequences set forth in SEQ ID NO:1 or SEQ ID NO:5 are also provided.

The specification describes an isolated nucleic acid from a methylotrophic yeast comprising an *FLD* promoter. The promoter is located upstream from the translational start codon of an *FLD* gene having a coding sequence with a sequence homology of about 70% to about 85% when compared to the nucleotide sequence of the *FLD* coding sequence set forth in SEQ ID NO:5. Also described is an isolated nucleic acid from a methylotrophic yeast comprising an *FLD* promoter from an *FLD* gene having a coding sequence with a sequence homology of about 85% to about 95% when compared to the nucleotide sequence of the *FLD* coding sequence set forth in SEQ ID NO:5. The promoter is from an *FLD* gene having a coding sequence with a sequence homology of greater than about 95% when compared to the nucleotide sequence of the *FLD* coding sequence set forth in SEQ ID NO:5. Particularly exemplified is a *Pichia pastoris FLD1* promoter comprising the sequence set forth in- SEQ ID NO:3.

Also described in the specification is an isolated nucleic acid comprising an *FLD* 3' termination sequence from a methylotrophic yeast. The 3' termination sequence is located downstream from the translational stop codon of an *FLD* gene having a coding sequence with a sequence homology of at about 70% to about 85% when compared to the nucleotide sequence of the *FLD* coding sequence set forth in SEQ ID NO:5. Also described, is an isolated nucleic acid comprising an *FLD* 3' termination sequence from a gene having a coding sequence with a sequence homology of at about 85% to about 95% when compared to the nucleotide sequence of the *FLD* coding sequence set forth in SEQ ID NO:5. Also described is an isolated nucleic acid comprising an *FLD* 3' termination sequence from a gene having a coding sequence with a sequence homology of greater than about 95% when compared to the sequence set forth in SEQ ID NO:5.

Also described are isolated nucleic acids comprising an *FLD* gene wherein said *FLD* gene encodes a product having an amino acid sequence identity of about 30% to about 49%, or about 50% to about 90% , or greater than about 90% when compared to the amino acid sequence as set forth in SEQ ID NO:2.

In addition, the specification also describes an isolated nucleic acid comprising at least one of a promoter, coding sequence or 3' termination sequence from an *FLD* gene wherein said *FLD* gene encodes a product having an amino acid sequence identity of about 30% to about 49% , or about 50% to about 90%, or greater than about 90% when compared to the amino acid sequence as set forth in SEQ ID NO:2.

In addition, the present invention provides expression cassettes, vectors and host cells comprising the subject isolated nucleic acids.

Also described in the specification is a method for directing expression of a heterologous gene in a methylotrophic yeast. The method comprises introducing into a methylotrophic yeast cell an isolated nucleic acid comprising an *FLD* promoter isolated from a methylotrophic yeast, said promoter operably linked at its 3' end to the 5' end of a heterologous gene, said heterologous gene operably linked at its 3' end to the 5' end of a termination sequence which functions in a methylotrophic yeast. The methylotrophic yeast cells are grown in a medium having a suitable carbon source such as glycerol or glucose and having a suitable nitrogen source such as an ammonium salt or ammonium hydroxide. After the carbon or nitrogen source is depleted, expression of said heterologous gene is induced by addition of methanol or methylamine or both methanol and methylamine. Expression may also be induced by the addition of formaldehyde, formate, or a methylated amine.

A method for selecting a formaldehyde resistant host cell is also described in the specification. The method comprises transforming a methylotrophic yeast cell with a vector comprising an *FLD* gene, said *FLD* gene operably linked at its 5' end to an *FLD* promoter or a heterologous promoter which functions in said yeast cell, said *FLD* gene operably linked on its 3' end to a 3' termination sequence which functions in said yeast cell. Host cells are grown in the presence of formaldehyde and a yeast cell which grows in the presence of formaldehyde is selected.

The specification also describes a strain of methylotrophic yeast which is defective in an *FLD* gene (*fld*) such as *Pichia pastoris* GS241 (*fld1-1*). Also provided is a strain of methylotrophic yeast which is defective in an *FLD* gene and auxotrophic for another biosynthetic gene.

### BRIEF DESCRIPTION OF THE DRAWINGS

Figure 1 provides physical maps of selected plasmids pHW018, pSS050, pK321, and pSS040.
Figure 2 is a restriction enzyme map of the *FLD1 gene*-containing vector pYG1.
Figure 3A shows exon analysis of the *FLD1* gene. A diagram of the expected products from PCR of unspliced (genomic) and spliced (cDNA) DNAs is indicated. Locations of the hybridized primers used in the PCR reactions are shown as convergent arrows.
Figure 3B is an electrophoregram of PCR and RT-PCR reaction products. PCR reactions were performed with the following: lane 1, genomic DNA template plus both primers; lane 2, cDNA template plus both primers; lane 3, cDNA template plus 5' primer only; lane 4, cDNA template plus 3' primer only; lane 5, both primers without DNA template. Flanking marker bands are denoted in base pairs.
Figure 4 is the nucleotide and deduced amino acid sequences of *P. pastoris FLD1* gene and its product.
Figure 5 is a comparison of the predicted amino acid sequences of *P. pastoris* and *C. maltosa* FLD proteins. Sequences were aligned using PC gene software. The character "*" between sequences indicates residues that are identical. The character "." indicates similar residues. Similar residues are defined as: A,S,T; D,E; N,Q; R,K; I,L,M,V; F,Y,W.
Figure 6 graphically depicts thermal stability of formaldehyde dehydrogenase activities in *P. pastoris* strains transformed with putative *FLD1* genes from *P. pastoris* and *H. polymorpha*. Strains shown are: wild-type *P. pastoris* (■); wild-type *H. polymorpha* (●); *P. pastoris* MS105 (pYG1) (□); and *P. pastoris* MS105 (pYG2) (○).
Figure 7A is a restriction map of the *Pichia pastoris FLD1* gene.
Figure 8 is a restriction map of *P*_{*FLD1*}.
Figure 9 is a restriction enzyme map of the *Hansenula polymorpha FLD* gene-containing vector pYG2.
Figure 10 is a restriction map of *an H. polymorpha* DNA fragment containing the *FLD* gene.
Figure 11 is a Southern blot showing genomic DNA from *H. polymorpha* digested with either BglII(B2)(lanes 1-3) or ClaI (C)(lanes 4-6) and hybridized with the following probes: pYG2 (lanes 1 and 4), pYM8(lanes 2 and 5), or pYG1 (lanes 3 and 6).

### DETAILED DESCRIPTION OF THE INVENTION

The present invention is directed to isolated nucleic acid sequences comprising formaldehyde dehydrogenase genes (*FLD*) from methylotrophic yeasts. The product of the *FLD* gene, formaldehyde dehydrogenase, confers resistance to formaldehyde. In one aspect of the invention, an *FLD* coding sequence may be used with its own 5' and 3' regulatory region or with a heterologous 5' and 3' regulatory region in order to function as a selectable marker in a methylotrophic yeast cell. The subject *FLD* coding sequences are therefore advantageous when use of antibiotic resistance genes as selectable markers is to be avoided.

In accordance with the present invention, a subject *FLD* gene can be used as a selectable marker that, like *Zeo*^{*R*}, can be selected for independent of the genotype of the *P*. *pastoris* strain and, like *Zeo*^{*R*} and *G418*^{*R*}, can be used to directly select strains that receive multiple copies of an expression vector. However, unlike *Zeo*^{*R*} and *G418*^{*R*}*,* the *P. pastoris PLD1* gene is native to *P. pastoris* and does not confer resistance to an antibiotic.

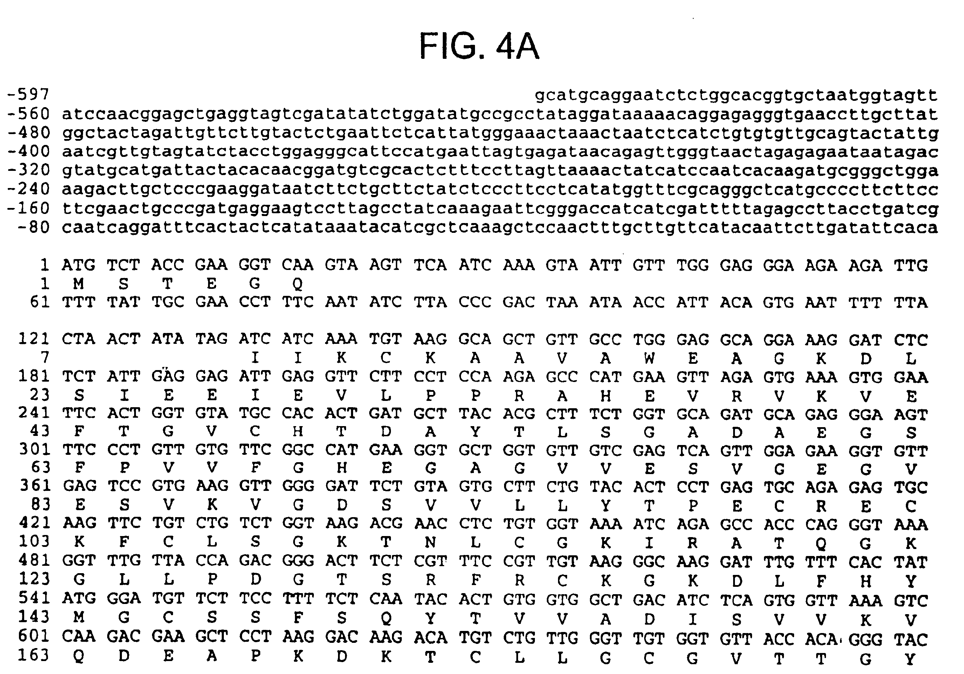

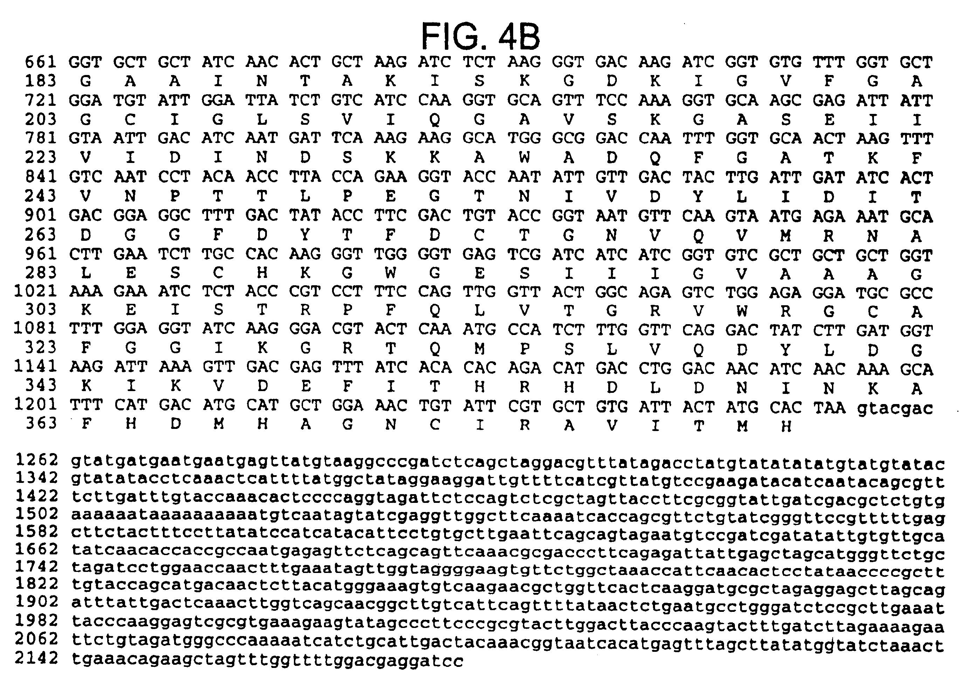

In one aspect of the present invention, there are provided *FLD* genes from *Pichia pastoris* and *Hansenula polymorpha* having the restriction maps set forth in Figures 7 and the cross hatched region of Figure 10, respectively. *FLD* expression in response to methanol or methylamine is controlled at the transcriptional level. The *FLD* gene from *Pichia pastoris* (*FLD1*) can be further described in terms of its nucleotide sequence which sequence is set forth in Figure 4 (SEQ ID NO:1). The nucleotide sequence of the coding region of the *FLD1* gene is set forth in SEQ ID NO:5.

In another aspect of the invention, there are provided inducible 5' regulatory regions from *FLD* genes (used herein interchangeably with *"FLD* promoters"), isolated from methylotrophic yeasts which 5' regulatory regions are useful for efficient expression of heterologous genes in cells of a methylotrophic yeast. The subject *FLD* 5' regulatory regions are strongly and independently induced by different carbon and/or energy sources such as methanol, formaldehyde, and formate. Neither formaldehyde nor formate are carbon sources in a true sense since *Pichia pastoris* does not utilize carbon from such compounds, but only obtains energy from their oxidation. The subject *FLD* 5' regulatory regions are also strongly and independently induced by different nitrogen sources such as methylamine, choline, and other methylated amines. Thus for example, the *Pichia pastoris FLD1* promoter is strongly and independently induced by either methanol as sole carbon source (with ammonium hydroxide or an ammonium salt as nitrogen source) or methylamine as sole nitrogen source (with a carbon sugar as carbon source). Examples of non-inducing nitrogen sources include ammonium sulfate, ammonium nitrate, ammonium chloride and ammonium hydroxide. Examples of non-inducing carbon sources include glycerol and glucose.

Accordingly, the present invention provides an isolated nucleic acid molecule comprising about 600 base pairs or more of nucleotide sequence located upstream from the translational start codon of an *FLD* gene from a methylotrophic yeast. Particularly exemplified is the promoter from the *Pichia pastoris FLD* gene *(FLD1)* having the restriction map illustrated in Figure 8. In a preferred embodiment, the *FLD1* gene promoter has the nucleotide sequence as set forth as SEQ ID NO:3. Also exemplified is the *FLD* promoter from *Hansenula polymorpha* having the restriction sites indicated in the cross hatched portion of Figure 10.

The specification also describes *FLD* 3' termination sequences from methylotrophic yeasts. There is an isolated nucleic acid comprising about 300 nucleotides or more of sequence located downstream from the translational stop codon of an *FLD* gene. For example, the 3' termination sequence may comprise nucleotides 1255-1555 of Figure 4 (SEQ ID NO:4). The 3' termination sequence is from the *Hansenula polymorpha FLD* gene which gene is shown as the cross hatched area in Figure 10.

Modifications to the *FLD1* promoter as set forth in SEQ ID NO:3, which maintain the characteristic property of promoting expression by either methanol, formaldehyde, or formate induction or by methylamine, choline or other methylated amine induction, are within the scope of the present invention. Modifications to the 3' termination sequence as set forth in SEQ ID NO:4, which maintain the characteristic property of stabilizing mRNA transcription products of a gene are also described. Similarly, modifications to the *Pichia pastoris FLD1* coding sequence (Figure 4, SEQ ID NO:5) which maintain the characteristic property of coding for a biologically active formaldehyde dehydrogenase are within the scope of the present invention. Such modifications include insertions, deletions and substitutions of one or more nucleotides.

The specification also describes methylotrophic yeast strains which are defective in the *FLD* gene, i.e., *fld* mutants. Such strains may be generated by exposing methylotrophic yeast cells to a mutagen such as nitrosoguanidine and screening for strains unable to utilize methanol as sole carbon source and methylamine as sole nitrogen source. Complementation and other genetic techniques may then be used to confirm that a methylotrophic yeast strain is an *fld* mutant, *a Pichia pastoris fld* strain is described and designated GS241 (*fld1-1*). An *fld* mutant methylotrophic yeast strain may be crossed to another strain which is an auxotrophic mutant for a biosynthetic gene or which has a different selectable marker. For example, a *Pichia pastoris* yeast strain is described which is methanol utilization defective (Mut⁻) and auxotrophic for histidine (His⁻), designated MS105 (*fld1-1 his4*).

An *FLD* gene may be isolated from a methylotrophic yeast using classic functional complementation techniques. Briefly stated, a genomic library of DNA from a methylotrophic yeast is cloned into a vector which replicates in a methylotrophic yeast. The vectors are used to transform a methylotrophic yeast which is an *fld* mutant. Cells which grow in the presence of methanol (or any of the above-described inducing agents) are selected as having a functional *FLD* gene from the genomic library. The vector is isolated from the complemented yeast cells and restriction mapped. Fragments of the vector insert may be subcloned and used to transform an *fld* mutant and a smaller fragment which still complements the *fld* mutant isolated. The insert of this vector may be sequenced and the *FLD* gene open reading frame (ORF) identified. As described in Examples 2 and 3, both the Pichia *pastoris FLD*1 gene and the *Hansenula polymorpha FLD* gene were isolated by functional complementation.

Nucleic acid molecules corresponding to coding sequences, promoters or 3' termination sequences of an *FLD* gene of a methylotrophic yeast may also be obtained by using the entire *FLD1* gene, the entire coding sequence of the *FLD1* gene, or portions of the *FLD1* coding sequence (including fragments and oligonucleotides) as a probe and hybridizing with a nucleic acid molecule(s) from a methylotrophic yeast. Nucleic acid molecules hybridizing to the *Pichia pastoris* entire *FLD* gene, (SEQ ID NO:1), or to the *FLD* coding sequence (Figure 4, SEQ ID NO:5) or portion of the nucleotide sequence set forth in SEQ ID NO:5, can be isolated, e.g., from genomic libraries by techniques well known in the art. Methods considered useful in obtaining genomic DNA sequences corresponding to the *Pichia pastoris FLD* gene of the present invention by screening a genomic library are provided in Sambrook et al. (1989), *Molecular Cloning: A Laboratory Manual,* Cold Spring Harbor, New York, for example, or any of the myriad of laboratory manuals on recombinant DNA technology that are widely available.

A subject *FLD* gene can be derived from restriction endonuclease digestion of isolated *FLD* genomic clones. Thus, for example, the known nucleotide or amino acid sequence of the Pichia *pastoris FLD1* gene (Figure 4, SEQ ID NOs:1 and 2) is aligned to the nucleic acid or deduced amino acid sequence of an isolated putative *FLD* genomic clone and the 5' regulatory sequence (i.e., sequence upstream from the translational start codon of the coding region), coding sequence, and 3' regulatory sequence (i.e., sequence downstream from the translational stop codon of the coding region) of the isolated *FLD* genomic clone located.

A subject *FLD* promoter, 3' termination sequence or coding sequence may be generated from genomic clones having excess 5' flanking sequence, excess coding sequence, or excess 3' flanking sequence by e.g., *in vitro* mutagenesis. *In vitro* mutagenesis is helpful for introducing convenient restriction sites. There are various commercially available kits particularly suited for this application such as the T7-Gen *in vitro* Mutagenesis Kit (USB, Cleveland, OH) and the QuikChange Site Directed Mutagenesis Kit (Stratagene, San Diego, CA). Alternatively, PCR primers can be defined to allow direct amplification of a subject *FLD* promoter, coding sequence and 3' termination sequence.

Using the same methodologies, the ordinarily skilled artisan can generate one or more deletion fragments of the *FLD1* promoter as set forth in SEQ ID NO:3. Any and all deletion fragments which comprise a contiguous portion of the nucleotide sequence set forth in SEQ ID NO:3 and which retain the capacity to promote expression by either methanol, formaldehyde, or formate induction or else which retain the capacity to promote expression by either methylamine, choline or other methylated amine induction are contemplated by the present invention. Similarly, any and all deletion fragments which comprise a contiguous portion of the sequence set forth in SEQ ID Nos:4 and 5 and which retain the capacity to stabilize mRNA transcription products of a gene or retain the capacity to code for a biologically active *FLD,* respectively, are described.

In addition to the *Pichia pastoris FLD1* promoter which nucleotide sequence is set forth as nucleotides -537 to -1 in Figure 4 (SEQ ID NO:3), the present invention is directed to other promoter sequences which correspond to *FLD* genes in other methylotrophic yeasts. As defined herein, such related sequences which promote expression by methanol, formaldehyde, or formate induction or else which promote expression by either methylamine, choline or other methylated amine induction, may be described in terms of their location upstream from the translational start codon of an *FLD* coding sequence, which coding sequence is described in terms of percent homology on a nucleotide level to the nucleotide coding sequence as set forth in Figure 4 (SEQ ID NO:5) .

Alternatively, *FLD* coding sequences from methylotrophic yeasts may be defined in terms of their ability to hybridize to the exemplified Pichia *pastoris FLD1* gene (SEQ ID NO:1) or *FLD1* coding sequence (SEQ ID NO:5) under low stringency hybridization conditions. The present invention therefore contemplates nucleic acid sequences isolated from a methylotrophic yeast comprising a promoter, coding region or 3' termination sequence corresponding to an *FLD* gene which coding region of such *FLD* gene hybridizes under low stringency conditions to the *FLD* gene nucleic acid sequence as set forth in SEQ ID Nos:1 or 5, or sequences complementary to the sequences set forth in SEQ ID NOs:1 or 5. The promoter, coding region or 3' termination sequences of an *FLD* gene which coding region hybridizes to a sequence as set forth in SEQ ID NOs:1 or 5, may differ in one or more nucleotide positions in comparison with SEQ ID NOs: 1 through 5 as long as such coding sequence from an *FLD* gene codes for a biologically active *FLD,* or as long as such *FLD* promoter is independently induced by either methanol, formaldehyde, or formate as energy source or by methylamine, choline or other methylated amine as sole nitrogen source. In addition, a subject 3' termination sequence may differ in one or more nucleotide positions in comparison to SEQ ID NO:4 as long as such 3' termination sequence retains the capacity to stabilize mRNA transcripts when operably linked to a coding sequence.

By "hybridizing" it is meant that such nucleic acid molecules hybridize under conventional hybridization conditions, such as described by, e.g., Sambrook (Molecular Cloning; A Laboratory Manual, 2nd Edition, Cold Spring Harbor Laboratory Press, Cold Spring Harbor, NY (1989)).

*FLD* genes (genomic sequences),and *FLD* coding sequences from methylotrophic yeasts may be identified by hybridization to the coding region or portions thereof of *FLD1,* (SEQ ID NOs: 1 and 5, as well as the complementary sequences to SEQ ID NOS: 1 AND 5) using conventional hybridization conditions. Preferably, low hybridization conditions are used such as 30% formamide at 37° C followed by washing in 1X SSC at room temperature and 1X SSC at 60° C. Putative *FLD* genes ranging in size from about 2 kb to about 3.5 kb or about 2.5 kb to about 3.5 kb which hybridize to SEQ ID NOs: 1 or 5 under low stringency conditions may be further characterized by restriction mapping and sequencing. Using the *FLD1* gene in the plasmid pYG1 as a probe and hybridizing under such low stringency conditions, the *H. polymorpha FLD* gene may be identified. *See* Example 3.

*FLD* promoters and 3' termination sequences may also be defined by the ability of the corresponding coding sequence of the *FLD* gene (from which the promoter or 3' termination sequence is derived), to hybridize under low stringency conditions to the coding sequence set forth in Figure 4 (SEQ ID NOs:1 and 5), as well as the complementary sequences to SEQ ID NOs:1 and 5.

*FLD* structural genes, promoter fragments and terminator sequences of the present invention may also be described in terms of percent homology on a nucleotide level to the nucleotide sequence provided herein. There are a number of computer programs that compare and align nucleic acid sequences which one skilled in the art may use for purposes of determining sequence homologies. For example, the PC/Gene program may be used (Release 6.6, IntelliGenetics, Inc., Mountainview, Ca.) with an open gap cost of 15 and a unit gap cost of 10.

As used herein, a sequence homology percentage value includes not only the percent homology of an isolated nucleic acid when compared to the single strand sequence set forth in a particular SEQ ID NO., but also includes the percent homology of an isolated nucleic acid when compared to the complementary strand of the single strand sequence set forth in the particular SEQ ID NO., such as SEQ ID NO:5.

Thus, using a computer program such as the PC/Gene program with the parameters set as described above, the subject isolated nucleic acids may be described as follows: an isolated nucleic acid comprising an *FLD* gene from a methylotrophic yeast which is independently inducible by either methanol as sole carbon source or methylamine as sole nitrogen source and having a coding sequence with a sequence homology of about 70% to about 85% when compared to the nucleotide sequence of the *FLD* gene as set forth in SEQ ID NO:5;
an isolated nucleic acid comprising an *FLD* gene which is independently inducible by either methanol as sole carbon source or methylamine as sole nitrogen source has a coding sequence with a sequence homology of about 85% to about 95% when compared to the coding sequence of the *FLD* gene as set forth in SEQ ID NO:5;
an isolated nucleic acid comprisirig an *FLD* gene which is independently inducible by either methanol as sole carbon source or methylamine as sole nitrogen source has a coding sequence with a sequence homology of greater than about 95% when compared to the sequence of the *FLD* coding region as set forth in SEQ ID NO:5;
an isolated nucleic acid comprising a promoter from an *FLD* gene which is independently inducible by either methanol as sole carbon source or methylamine as sole nitrogen source comprises approximately 600 bases pairs or more of nucleotide sequence located upstream (5') from the translation start codon of an *FLD* gene, whose coding sequence has a sequence homology of about 70% to about 85% when compared to the nucleotide sequence of the *FLD* coding sequence as set forth in SEQ ID NO:5; an isolated nucleic acid comprising a promoter from an *FLD* gene which is independently inducible by either methanol as sole carbon source or methylamine as sole nitrogen source comprises approximately 600 bases pairs or more of nucleotide sequence located upstream (5')from the translation start codon of an *FLD* gene, whose coding sequence has a sequence homology of about 85% to about 95% when compared to the nucleotide sequence of the *FLD* coding sequence as set forth in SEQ ID NO:5;
an isolated nucleic acid comprising a promoter from an *FLD* gene which is independently inducible by either methanol as sole carbon source or methylamine as sole nitrogen source comprises approximately 600 bases pairs or more of nucleotide sequence located upstream (5') from the translation start codon of an *FLD* gene*,* whose coding sequence has a sequence homology of greater than about 95% when compared to the nucleotide sequence of the *FLD* coding sequence as set forth in SEQ ID NO:5. With respect to any of the above-described promoters, preferably, a promoter comprises approximately 600 base pairs or more of nucleotide sequence located immediately upstream (5') to the translational start codon of an *FLD* gene.

The specification describes an isolated nucleic acid comprising an *FLD* 3' termination sequence from a methylotrophic yeast comprises approximately 300 base pairs or more of nucleotide sequence located downstream (3') from the translational stop codon of an *FLD* gene, whose coding sequence has a sequence homology of about 70% to about 85% when compared to the nucleotide sequence of the *FLD* coding sequence as set forth in SEQ ID NO:5. Also described is, an isolated nucleic acid comprising an *FLD* 3' termination sequence from a methylotrophic yeast comprises approximately 300 base pairs or more of nucleotide sequence downstream (3') from the translational stop codon of an *FLD* gene, whose coding sequence has a sequence homology of about 85% to about 95% when compared to the nucleotide sequence of the *FLD* coding sequence as set forth in SEQ ID NO:5. Also described is an isolated nucleic acid comprising an *FLD* 3' termination sequence from a methylotrophic yeast comprises approximately 300 base pairs or more of nucleotide sequence located downstream (3') from the translational stop codon of an *FLD* gene, whose coding sequence has a sequence homology of greater than about 95% when compared to the nucleotide sequence of the *FLD* coding sequence as set forth in SEQ ID NO:5. With respect to any of the above-described 3' termination sequences, preferably a 3' termination sequence comprises approximately 300 base pairs or more of nucleotide sequence located immediately downstream (3') to the translational stop codon of an *FLD* gene.

In addition to the aforementioned nucleic acid sequences, the present invention contemplates isolated nucleic acids comprising promoters, coding sequences and 3' termination sequences from an *FLD* gene whose product has an amino acid sequence identity of about 30% to about 49% when compared to the amino acid sequence set forth in Figure 4 (SEQ ID NO:2). Also descrised is, an isolated nucleic acid comprising a promoter, coding sequence or 3' termination sequence from an *FLD* gene is from an *FLD* gene whose product has an amino acid sequence identity of about 50% to about 90% when compared to the amino acid sequence set forth in Figure 4 (SEQ ID NO:2). Also described is an isolated nucleic acid comprising a promoter, coding sequence or 3' termination sequence from an *FLD* gene is from an *FLD* gene whose product has an amino acid sequence identity of greater than about 90% when compared to the amino acid sequence set forth in Figure 4 (SEQ ID NO:2) . In a most preferred embodiment, an isolated nucleic acid comprising a promoter, coding sequence or 3' termination sequence from an *FLD* gene is from an *FLD* gene whose product has an amino acid sequence as set forth in Figure 4 (SEQ ID NO:2).

An entire *FLD* gene (i.e., a genomic sequence comprising *FLD* coding sequence operably linked to the native *FLD* promoter and native 3' termination sequence) may also be described by the sequence identity of the product of the coding region. Thus, an *FLD* gene is described wherein the amino acid sequence of the product of the *FLD* gene has a sequence identity of about 30% to about 49% when compared to the amino acid sequence set forth in Figure 4 (SEQ ID NO:2). In a preferred embodiment, there is provided an *FLD* gene wherein the amino acid sequence of the product of the *FLD* gene has a sequence identity of about 50% to about 90% when compared to the amino acid sequence set forth in Figure 4 (SEQ ID NO:2). Also described is an isolated nucleic acid comprising an *FLD* gene codes for a product having an amino acid sequence with a sequence identity of greater than about 90% when compared to the amino acid sequence set forth in Figure 4 (SEQ ID NO:2). In a most preferred embodiment, an *FLD* gene codes for a product having an amino acid sequence as set forth in Figure 4 (SEQ ID NO:2).

For purposes of determining the degree of sequence identity between a putative *FLD* amino acid sequence from a methylotrophic yeast and the *FLD* amino acid sequence provided herewith as SEQ ID NO:2, the BLAST 2.0 program (GenBank, National Center for Biotechnology Information) may be used with all parameters set to default parameters.

To determine the nucleotide sequence of an isolated *FLD* nucleic acid molecule, any of the various well known techniques may be used. For example, restriction fragments containing an *FLD* gene from *Pichia pastoris* or other methylotrophic yeast can be subcloned into the polylinker site of a vector such as pBluescript (Stratagene). These pBluescript subclones can then be sequenced by the double-stranded dideoxy method (Chen et al. (1985) *DNA*, 4; 165).

5' regulatory sequence, coding sequence, and 3' termination sequence from a methylotrophic yeast *FLD* gene which correspond to *Pichia pastoris FLD* gene sequences may also be isolated by applying a nucleic acid amplification technique such as the polymerase chain reaction (PCR) using as primers oligonucleotides derived from sequences set forth in SEQ ID NOs:1, 3, 4, and 5.

Confirmation of independent inducibility of an *FLD* promoter (including modifications or deletion fragments thereof) from a methylotrophic yeast, can be accomplished by construction of transcriptional and/or translational fusions of specific sequences with the coding sequences of a heterologous gene, transfer of the chimeric gene into an appropriate host, and detection of the expression of the heterologous gene. The assay used to detect expression depends upon the nature of the heterologous sequence. For example, reporter genes, exemplified by β-lactamase (β-lac), β-galactosidase (β-gal), luciferase and chloramphenicol acetyltransferase (CAT) are commonly used to assess transcriptional and translational competence of chimeric constructions. Standard assays are available to sensitively detect the reporter enzyme in a transformed host cell.

An FLD promoter, 3' termination sequence and isolated fragments thereof, are useful in the construction of expression cassettes (also termed herein "chimeric genes") and expression vectors for the expression of heterologous proteins in a methylotrophic host cell. As used herein, "heterologous protein" or "heterologous polypeptide" refers to any protein or polypeptide other than formaldehyde dehydrogenase. As used herein, "heterologous gene" means a gene other than *FLD.*

As used herein, the term "cassette" refers to a nucleotide sequence capable of expressing a particular gene if said gene is inserted so as to be operably linked to one or more regulatory sequences present in the nucleotide sequence. Thus, for example, the expression cassette may comprise a heterologous gene which is desired to be expressed through methanol or methylamine induction. The expression cassettes and expression vectors of the present invention are therefore useful for promoting expression of any number of heterologous genes upon methanol or methylamine induction.

Some examples of heterologous genes for expression of foreign proteins under control of the subject FLD promoter and for use in the expression cassettes and vectors of the present invention include human serum albumin, invertase, bovine lysozyme, human EGF, mouse EGF, aprotinin, Kunitz protease inhibitor, Hepatitis B surface antigen, tumor necrosis factor, tetanus toxin fragment C, pertussis antigen P69, streptokinase, β-galactosidase, and *Bacillus sp.* crystal protein toxin. For a list of other useful proteins which may be expressed in *Pichia pastoris, see* Higgins, D.R. and Cregg, J.M. (1998) *Methods in Molecular Biology: Pichia Protocols.* Humana Press, Totowa, New Jersey, Chapter 17, pp. 249-261. Any and all coding sequences are contemplated as heterologous genes for use in the expression cassettes and expression vectors of the present invention.

The expression cassettes of the present invention comprise in the 5' to 3' direction an *FLD* promoter operably linked a nucleotide sequence coding for a heterologous gene. In a preferred embodiment, the coding sequence for a heterologous gene is further operably linked at its 3' end to a 3' termination sequence. If desired, additional regulatory elements from genes other than *FLD* or parts of such elements sufficient to cause expression resulting in production of an effective amount of the polypeptide encoded by the heterologous gene are included in the chimeric constructs. For example, signal sequences coding for transit peptides may be used when secretion of a product of a heterologous gene is desired. Such sequences are widely known, readily available and include *Saccharomyces cerevisiae* alpha mating factor pre pro (αmf), *Pichia pastoris* acid phosphatase (PHO1) signal sequence and the native signal sequence from the protein encoding heterologous gene.

The expression cassette may be inserted into a microorganism host via a vector such as a circular plasmid or linear site-specific integrative vector. The term "operably linked" refers to a juxtaposition wherein the FLD promoter , structural gene, and 3' termination sequence are linked and configured so as to perform their normal function. 3' termination sequences are sequences 3' to the stop codon of a structural gene which function to stabilize the mRNA transcription product of the gene to which the sequence is operably linked, such as sequences which elicit polyadenylation. 3' termination sequences may be obtained from *Pichia* or *Hansenula polymorpha* or other methylotrophic yeast. Examples of *Pichia pastoris* 3' termination sequences useful for the practice of the present invention include termination sequences from the *AOX1* gene, *p40* gene, *HIS4* gene and *FLD1* gene.

In accordance with the present invention, the *Pichia pastoris FLD1* gene, the Hansenula *polymorpha FLD* gene, and other *FLD* genes isolated from methylotrophic yeasts, may be used as selectable markers in host cells. Either the entire *FLD* gene, including the native 5' and 3' regulatory sequences or the *FLD* coding region operably linked to 5' and 3' regulatory regions other than that of an *FLD* gene may be used.

The isolated nucleic acids comprising an *FLD* promoter, *FLD* coding sequence and/or *FLD* 3' termination sequence, the subject expression cassettes comprising such isolated nucleic acids as well as an entire *FLD* gene (genomic sequence) or *FLD* coding sequence operably linked to 5' and 3' regulatory regions other than that of an *FLD* gene, may be inserted into a vector such as a plasmid. The vector preferably contains a selectable marker gene which functions in a methylotrophic yeast. The selectable marker may be any gene which confers a selectable phenotype upon a methylotrophic yeast and allows such yeast to be identified and selected from untransformed cells. The selectable marker system may include an auxotrophic mutant *Pichia pastoris* host strain and a wild type gene which complements the host's defect. Examples of such systems include the *Saccharomyces cerevisiae* or *Pichia pastoris HIS4* gene which may be used to complement *his4 Pichia* strains, or the *S. cerevisiae* or *Pichia pastoris* ARG4 gene which may be used to complement *Pichia pastoris arg* mutants. Other selectable marker genes which function in *Pichia pastoris* include the *Zeo*^{*R*} gene, the *G418*^{*R*} gene, and of course, the *FLD* genes of the present invention.

The vectors of the present invention may also contain selectable marker genes which function in bacteria. The added bacterial selectable marker permits amplification of the vector in bacterial host cells. Examples of bacterial selectable marker genes include ampicillin resistance (Amp^{r}), tetracycline resistance (Tet^{r}), neomycin resistance, hygromycin resistance, and zeocin resistance (*Zeo*^{*R*}).

In addition, the vectors of the present invention may include sequences responsible for replication and extrachromosomal maintenance in bacteria such as *E. coli.* The use of such sequences allows for amplification of the vector in bacteria and thus production of large amounts of the vector DNA. Examples of bacterial origins of replication include colisin, col D1, col E1 and others known to skilled artisans.

The vectors of the present invention may also contain an autonomous replication sequence (ARS) such as described in U.S. Patent No. 4,837,148, issued June 6, 1989 to James M. Cregg. The disclosure of U.S. Patent No. 4,837,148 is incorporated herein as if fully set forth. The autonomous replication sequences disclosed by Cregg provide a suitable means for maintaining plasmids in *Pichia pastoris*.

Alternatively, integrative vectors may be used rather than circular plasmids. Such integrative vectors are disclosed in U.S. Patent No. 4,882,279, issued November 21, 1989 to James M. Cregg. The '279 patent is also incorporated herein by reference as if fully set forth. Integrative vectors suitable for use with the subject promoters, 3' termination sequences, *FLD1* marker genes and expression cassettes comprise a serially arranged sequence of at least a first insertable DNA fragment, a selectable marker gene, and a second insertable DNA fragment. An expression cassette containing a heterologous structural gene is inserted in this vector between the first and second insertable DNA fragments whether before or after the marker gene. Alternatively, an expression cassette can be formed *in situ* if the *FLD* promoter is contained within one of the insertable fragments to which the structural gene may be operably linked.

The first and second insertable DNA fragments are each at least about 200 nucleotides in length and have nucleotides sequences which are homologous to portions of the genomic DNA of the species to be transformed. Insertable fragments may be as low as 50 nucleotides in length if a diploid strain of *Pichia pastoris* is used. The various components of the integrative vector are serially arranged forming a linear fragment of DNA such that the expression cassette and the selectable marker gene are positioned between the 3' end of the first insertable DNA fragment and the 5' end of the second insertable DNA fragment. The first and second insertable DNA fragments are oriented with respect to one another in the serially arranged linear fragment as they are oriented in the parent genome.

Nucleotide sequences useful as the first and second insertable DNA fragments are nucleotide sequences which are homologous with separate portions of the native genomic site at which genomic modification is to occur. For example, if genomic modification is to occur at the locus of the alcohol oxidase gene, the first and second insertable DNA fragments employed would be homologous to separate portions of the alcohol oxidase gene locus. Examples of nucleotide sequences which could be used as first and second insertable DNA fragments are deoxyribonucleotide sequences such as the *Pichia pastoris* alcohol oxidase (*AOX1*) gene, dihydroxyacetone synthase (*DAS1*) gene, *p40* gene and *HIS4* gene. The *AOX1* gene, *DAS1* gene, p40 gene, and *HIS4* genes are disclosed in U.S. Pat. Nos. 4,855,231, and 4,885242, both incorporated herein by reference. The designation DAS1 is equivalent to the DAS designation originally used in U.S. Pat. Nos. 4,855,231 and 4,885,242. The first insertable DNA fragment may contain a *FLD* promoter which *FLD* promoter is also part of the expression cassette. A second insertable DNA fragment may contain 3' flanking sequence starting about 300 base pairs downstream from the translational stop codon of an *FLD* gene.

The vectors and chimeric genes of the present invention can be constructed by standard techniques known to one of ordinary skill in the art and found, for example, in Sambrook et al. (1989) in Molecular Cloning: A Laboratory Manual, or any of a myriad of laboratory manuals on recombinant DNA technology that are widely available. A variety of strategies are available for ligating fragments of DNA, the choice of which depends on the nature of the termini of the DNA fragments and can be readily determined by the skilled artisan.

When the methylotrophic yeast host cells are transformed with a linear DNA fragment comprising a heterologous gene under the control of the FLD promoter, the expression cassette is integrated into the host cell genome by any of the gene replacement methods known in the art such as by one-step gene replacement. Rothstein, *1983 Methods Enzymol.* 101:202 and Cregg *et al.*, 1987 *Bio*/*Technology* 5:479. When the DNA vector is a circular plasmid, such plasmid may be linearized to facilitate integration and then integrated into the methylotrophic yeast genome at the same or different loci by addition. Cregg et al. (1985) *Mol. Cell. Biol.5* :3376.

The vectors of the present invention may be transformed into the cells of a methylotrophic yeast using known methods such as the spheroplast technique, described by Cregg et al. 1985, or the whole-cell lithium chloride yeast transformation system, Ito et al. *Agric. Biol. Chem. 48*:341, modified for use in *Pichia* as described in EP 312,934. Other published methods useful for transformation of the plasmids or linear vectors of the present invention include U.S. Patent No. 4,929,555 to Cregg and Barringer; Hinnen et al. (1978) *Proc. Nat.. Acad. Sci . 75*:1929; Ito et al. (1983) *J. Bacteriol.* 153:163; U.S. Patent No. 4,879,231 to D.W. Stroman et al; Sreekrishna et al. (1987) *Gene* 59:115. Electroporation and PEG1000 whole cell transformation procedures may also be used. Cregg and Russel (1985) *Methods in Molecular Biology: Pichia Protocols,* Chapter 3, Humana Press, Totowa, N.J., pp. 27-39.

In accordance with the present invention, host cells are provided which comprise the subject expression cassettes and expression vectors. The yeast host for transformation may be any suitable methylotrophic yeast. Suitable methylotrophic yeasts include but are not limited to yeast capable of growth on methanol such as yeasts of the genera *Candida, Hansenula, Torulopsis,* and *Pichia*. A list of species which are exemplary of this class of yeasts may be found in C. Anthony (1982), *The Biochemistry of Methylotrophs, 269. Pichia pastoris, Pichia methanolica, Pichia anomola, Hansenula polymorpha and Candida boidinii* are examples of methylotrophic yeasts useful in the practice of the present invention. Preferred methylotrophic yeasts are of the genus *Pichia*. Especially preferred are *Pichia pastoris* strains GS115 (NRRL Y-15851); GS190 (NRRL Y-18014) disclosed in U.S. Patent No. 4,818,700; and PPF1 (NRRL Y-18017) disclosed in U.S. Patent No. 4,812,405. Auxotrophic *Pichia pastoris* strains such as GS115, GS190 and PPF1 are advantageous to the practice of the present invention for their ease of selection. Wild type Pichia *pastoris* strains such as NRRL Y-11430 and NRRL Y-11431 may be employed with equal success if a suitable transforming marker gene is selected, such as the use of SUC2 to transform *Pichia pastoris* to a strain capable of growth on sucrose or if antibiotic resistance marker is employed, such as resistance to G418 and zeocin.

For the large-scale production in *Pichia pastoris* of heterologous proteins using the vectors of the present invention, a two-state, high cell-density, batch fermentation may be employed. During the first stage (growth stage), *Pichia* host cells may be cultured in defined minimal medium with a suitable carbon source such as glycerol or glucose, and a suitable nitrogen source such as ammonium sulfate, ammonium nitrate or other ammonium salt. Ammonium hydroxide may also be used. In this first stage, heterologous gene expression is repressed, which allows cell expansion and generation of cell mass. Once the repressing carbon or nitrogen source is depleted, either methanol or methylamine, or both, are added which initiates expression of the heterologous gene in the second stage (production stage). In accordance with the present invention, induction using both methanol and methylamine provides a synergistic effect. That is, levels of gene expression are higher when both methanol and methylamine are used to induce compared to when methanol alone or methylamine alone is used to induce.

Alternatively, gene expression may be induced using formaldehyde or formate as energy source or choline and other methylated amines as nitrogen source. If methanol is used to induce, it is used in a concentration of 1% or less. Very small amounts, down to almost nothing are all that is needed to induce expression. If formaldehyde is used to induce, an amount of about 10 mM to almost nothing is used, keeping in mind that formaldehyde is very toxic to *P. pastoris* in amounts of 10 mM or higher. Formate is also very toxic to *P*. *pastoris* in amounts greater than 100 mM. If methylamine, choline or other methylated amines are used to induce gene expression, an amount of 0.5% to almost nothing is used.

The host cells may be grown in the temperature range of about 35 degrees Centigrade (C) down to 4 degrees C. A preferred temperature for growth of cells is 30 degrees C. The pH range for growth of cells is 2.8 to 7.5 with a preferred ranged of 3.0 to 6.5. Conditions and methodologies for growth of methylotrophic yeast cells are thoroughly discussed in Higgins and Cregg (1998) *Methods in Molecular Biology: Pichia protocols,* Humana Press, Totowa, New Jersey, and are incorporated herewith as if fully set forth.

Transformed *Pichia pastoris* cells may be selected by using appropriate techniques including but not limited to culturing previously auxotrophic cells after transformation in the absence of the biochemical product required (due to the cell's auxotrophy), selection for and detection of a new phenotype ("methanol slow") or culturing in the presence of an antibiotic which is toxic to the yeast in the absence of a resistance gene contained in the transformant.

As discussed hereinbefore, a subject *FLD* gene may be used as a selectable marker to transform a methylotrophic yeast cell for purposes of direct selection for formaldehyde resistance. In addition, the Specification describes a method for direct selection of a transformed host cell which comprises introducing into a host cell a vector comprising an *FLD* coding sequence operably linked to an *FLD* promoter as defined herein or operably linked to a heterologous promoter. Optimally, the *FLD* coding sequence is also operably linked at its 3' end to a 3' termination sequence. Transformed host cells are grown in the presence of formaldehyde and resistant cells selected.

Levels of formaldehyde used to select for resistant cells will depend on the yeast strain used as a host cell, and the promoter used to drive expression of the *FLD* gene. For example, if a wild type *Pichia pastoris* strain and native *FLD* promoter are used, then a level of about 7mM formaldehyde is enough to allow for direct selection. If an *fld* mutant *Pichia pastoris* strain is used with either a native *FLD* promoter or a heterologous promoter (i.e., a promoter other than the *FLD* promoter), then a level of about 2 mM is enough to allow for direct selection.

Positive transformants may be characterized by Southern blot analysis (Sambrook et al. 1989) which is particularly useful for identifying the site of DNA integration. Northern analysis (Sambrook et al. 1989) may be used to confirm methanol-responsive and methylamine responsive gene expression. The product of the heterologous gene may also be assayed using well known methodologies and isolates which produce the desired gene product at the appropriate level identified. Immunoblotting using polyclonal or monoclonal antibodies to the product of the heterologous gene may also be used.

Another aspect of the present invention provides a method for directing expression of a heterologous gene in a methylotrophic yeast which comprises introducing into a methylotrophic yeast cell an isolated nucleic acid comprising an *FLD* promoter isolated from a methylotrophic yeast, which promoter is operably linked at its 3' end to the 5' end of a heterologous gene. Optimally, the heterologous gene is also operably linked at its 3' end to the 5' end of a 3' termination sequence which functions in a methylotrophic yeast. Such an isolated nucleic acid is preferably within a vector which replicates within a methylotrophic yeast or which integrates into the genome of a methylotrophic yeast as hereinbefore described. A methylotrophic yeast cell is transformed with the expression cassette or expression vector and then the cell is grown in a medium having sugar such as glycerol or glucose as carbon source and ammonium hydroxide, ammonium sulfate, ammonium nitrate, or other ammonium salt as nitrogen source. After the repressing carbon or nitrogen source is depleted, expression of the heterologous gene is induced by addition of methanol or methylamine. Alternatively, gene expression may be induced with formaldehyde or formate as energy source or choline and other methylated amines as nitrogen source. Routine methods are used to isolate the heterologous protein from the culture medium (if the heterologous protein is secreted from the host cells) or from the methylotrophic yeast cells (if the heterologous protein is not secreted).

The invention is further illustrated by the following specific examples which are not intended in any way to limit the scope of the invention.

### EXAMPLES

The strains, plasmids, and media employed in the following examples have the compositions given below:

The wild-type *P. pastoris* strain used was NRRL Y-11430. *P. pastoris f1d1* mutant strains were generated using nitrosoguanidine and were obtained through Dr. George Sperl of Phillips Petroleum Company (Bartlesville, OK, USA). The *Pichia pastoris fld1* strain GS241 (*fld1-1*) was deposited at the Northern Regional Research Center of the U.S. Department of Agriculture (NRRL), Peoria, Illinois on and assigned Accession No. . MS105, a *P. pastoris fld1 his4* strain, was constructed by crossing GS241 (*fld1-1*) with GS115 *(his4).* MS105 was also deposited at the NRRL on and assigned Accession No. . The plasmids pYG1 and pYG2 have been deposited in at the NRRL on and assigned Accession Nos. and respectively. The *Hansenula polymorpha* strain used was CBS4732. Bacterial recombinant DNA manipulations were performed in either *Escherichia coli* strain MC1061 or DH5α. Yeast strains were cultured in a rich YPD medium (1% yeast extract, 2% peptone, 0.4% glucose) or a minimal medium composed of 0.17% yeast nitrogen base without ammonium sulfate and amino acids, a carbon source (0.4% glucose or 0.5% methanol), and a nitrogen source (0.5% ammonium sulfate or 0.25% methylamine chloride). *E*. *coli* strains were cultured in Luria broth medium supplemented with either 100 µg/ml ampicillin or 50 µg/ml zeocin (Invitrogen Corporation, Carlsbad, CA, USA) as required.

### EXAMPLE 1

### Isolation of formaldehyde dehydrogenase-defective mutants of P. pastoris

As a first step in cloning the *P. pastoris* formaldehyde dehydrogenase gene *(FLD1),* mutants were sought that were specifically defective in FLD activity. Previous biochemical studies of methylotrophic yeasts indicated that FLD is involved in the metabolism of both methanol as carbon source and methylamine as nitrogen source (Zwart et al., 1983). To search for *P. pastoris fld1* mutants, nitrosoguanidine-mutagenized cultures were screened for strains that were unable to utilize methanol as carbon source and methylamine as nitrogen source. Complementation analysis and other classical genetic techniques were performed as described in Cregg and Russell (1998). Five mutants belonging to a single complementation group were identified.

These five strains were further examined by measuring the levels of activity of key methanol pathway enzymes in extracts prepared from methanol-induced cultures of each strain. These enzymes included: alcohol oxidase (AOX), catalase, dihydroxyacetone synthase, dihydroxyacetone kinase, FLD, and formate dehydrogenase. For enzyme assays, yeast strains were grown in shake flasks at 30°C in YNB medium (without amino acids and ammonium sulfate, DIFCO) using 0.5% methanol as carbon source and 0.5% ammonium sulfate as nitrogen source. Cultures were harvested in the late logarithmic phase, and cell-free extracts were prepared using glass beads as described in Waterham et al. (1996). The protein concentrations in cell-free extracts were determined using either the method of Bradford (1976) or the Pierce BCA protein assay kit (Rockford, IL) with bovine serum albumin as standard. Alcohol oxidase (van der Klei et al., 1990), catalase (Lück, 1963), dihydroxyacetone synthase (Waites and Quayle, 1981), dihydroxyacetone kinase (van Dijken et al., 1978), and formate dehydrogenase (van Dijken, 1976) activities were determined by published methods. Formaldehyde dehydrogenase activity was measured spectrophotometrically by following the rate of NADH formation at 340 nm in the presence of saturating amounts of formaldehyde, glutathione, and NAD as described by Schutte et al. (1976). Reaction mixtures contained 33 mM sodium phosphate buffer (pH 7.9-8.0), 2 mM glutathione, 1 mM NAD, 1 mM formaldehyde, and limiting amounts of enzyme in a final volume of 1.0 ml. The rate of absorbance change at 340 nm was followed for at least 2 min, and activities were calculated by using the constant ε = 6.22 cm²/nmol for NAD. Alcohol oxidase activities were expressed in µmol/mg/min, and formaldehyde dehydrogenase activities were expressed in µmol/mg/min. β-lactamase activity, expressed as nmol/mg/min, was assayed spectrophotometrically at 569 nm and 30°C in 25 mM Tris-HCl (pH 7.5) using 11.1 mM PADAC as substrate (extinction coefficient 44.403 cm⁻¹M⁻¹).

As shown in Table 1, growth of wt *P*. *pastoris* on methanol as sole carbon source and ammonium sulfate as sole nitrogen source specifically induced high levels of FLD activity (Table 1). Results were essentially the same for each of the five mutants and are shown in Table 1 for one of the mutant strains GS241. Each mutant contained significant levels of activity for all enzymes assayed except FLD which was undetectable. As controls, methanol-grown wild-type *P. pastoris* had normal levels of FLD activity, and methanol-induced cells of a *P. pastoris* strain that is deleted for its *AOX* genes and as a result cannot grow on methanol also contained substantial levels of FLD activity.

The phenotypic and biochemical characteristics of the mutants were consistent with the finding that they were specifically defective in the *P*. *pastoris FLD1* gene. One putative *fld1* strain, GS241 (*fld1-1*), was selected for all further manipulations.

### EXAMPLE 2

### Isolation and characterization of the P. pastoris FLD1 gene

To clone the putative *FLD1* gene by functional complementation, strain GS241 was first crossed to *P*. *pastoris* strain GS115 *(his4)* to obtain a derivative that was both methanol-utilization defective (Mut⁻) and auxotrophic for histidine (His⁻). One Mut⁻His⁻ strain that resulted from this cross, MS105 (*fld1-1 his4),* was then transformed with 5-10 µg of a *P. pastoris* genomic DNA library constructed in the *P. pastoris-E*. *coli* shuttle vector pYM8 using the spheroplast method (Cregg et al., 1985; Liu et al., 1995). The plasmid pYM8 is composed of the *Saccharomyces cerevisiae* histidinol dehydrogenase gene (*SHIS4*) and a *P. pastoris*-specific autonomous replication sequence (*PARS1*) inserted into *E. coli* plasmid pBR322. Approximately 50,000 library transformants were selected for His⁺ prototrophy on YND medium agar and resultant selected clones further selected on YNM plates for Mut⁺ phenotype. Total DNA was extracted from a pool of several hundred His⁺ Mut⁺ colonies and used to transform *E. coli.* One plasmid recovered from this process, pYG1, was able to retransform strain MS105 to both His⁺ and Mut⁺ and was examined further.

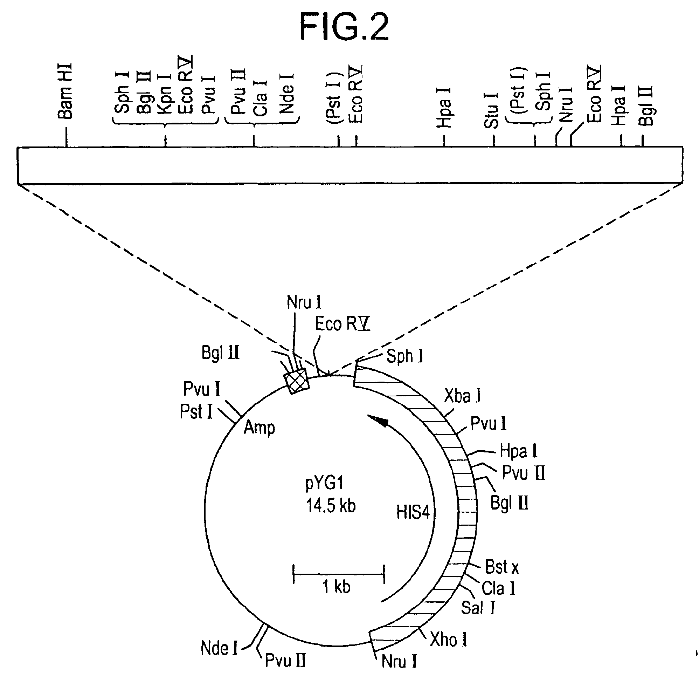

To determine the location of the putative *FLD1* gene on pYG1, the plasmid was restriction mapped, and selected fragments from the vector were subcloned and tested for the ability to complement strain MS105. Recombinant DNA methods were performed essentially as described in Sambrook et al. (1989). Oligonucleotides were synthesized and DNA sequencing was performed at the Oregon Regional Primate Research Center, Molecular Biology Core Facility (Beaverton, OR, USA). The plasmid was found to be 14.5 kb in size and to contain an insert of 6.8 kb (Fig. 2). A 2.7-kb *Sph*I-*Bam*HI fragment was found to be sufficient to complement the Mut⁻ defect in MS105 and was sequenced. The DNA sequence identified a long open reading frame (ORF) whose predicted product had strong similarity to other alcohol dehydrogenases. The sequence also suggested the possible presence of an intron near the 5' terminus of the gene.

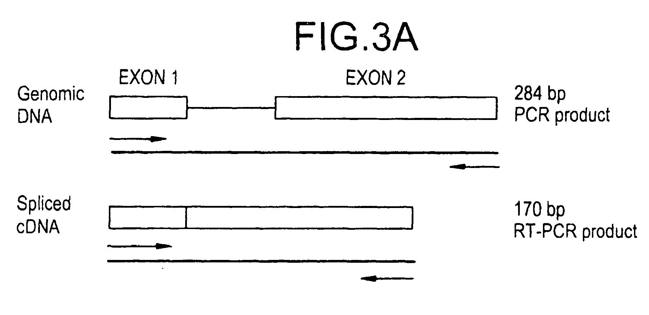

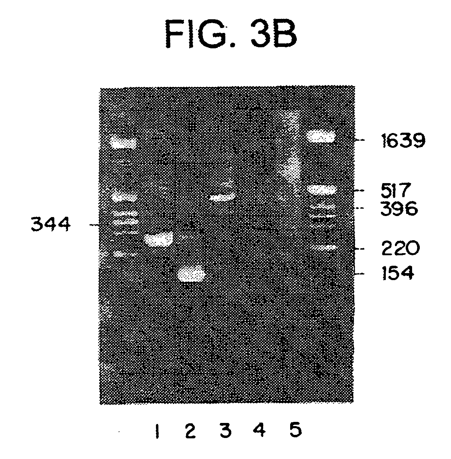

To confirm the presence of an intron, this region of the ORF was amplified from mRNA by the reverse transcriptase-polymerase chain reaction method (RT-PCR), and the size and sequence of the product was compared to that obtained by PCR of the genomic fragment on plasmid pYG1 (Fig. 3). PCR reactions were performed as described by Kramer and Coen (1995). Total *P. pastoris* RNA was isolated according to Schmitt et al. (1990). The RT-PCR reaction was performed as previously described (Frohman et al., 1988; Stewart et al., 1992) using the following oligonucleotide primers: 5' '-CACAATGTCTACCGAAGGTC-3 " (5' primer) and 5'-CCAGAAAGCGTGTAAGCATCAG-3' (3' primer).

Whereas the genomic product was 284 bp in length, the cDNA product was significantly shorter at 170 bp. Alignment of the cDNA and genomic sequences demonstrated that a segment of 114 bp that was present in the genomic DNA was absent from the cDNA. Furthermore, examination of the putative intron/exon junctions revealed typical yeast splice junctions (5' junction, 5'-GTAAGT-3 "; 3' junction, 5'-YAG-3' ') and branch point (5'-TACTAAC-3') (Domdey et al., 1984; Sasnauskas et al., 1992). A single intron is therefore present at this position in the ORF. Finally, Southern blots of selected restriction digests of wild-type genomic DNA, using a fragment from the ORF as hybridization probe, indicated that the *P. pastoris* genome contained only one copy of the gene.

The DNA and predicted amino acid sequences of the ORF are shown in Fig. 4. The sequence data are available from EMBL/GenBank/DDBJ under accession number AF066054. The ORF is 1,137 bp long and is predicted to encode a protein of 379 amino acids with a calculated molecular mass of 39,870. The intron begins at a position 18 bp (six amino acids) 3' of the A of the predicted methionine initiator ATG and is 114 bp in length. Northern blots of total RNA extracted from glucose- and methanol-grown wild-type *P. pastoris* cells, using a DNA fragment from the ORF region, showed a single mRNA species of approximately 1.3 kb that was present at high levels in methanol- but not glucose-grown cells (data not shown). Overall, the codon usage of the putative *FLD1* gene was typical of other highly expressed *P. pastoris* genes (Sreekrishna, 1993).

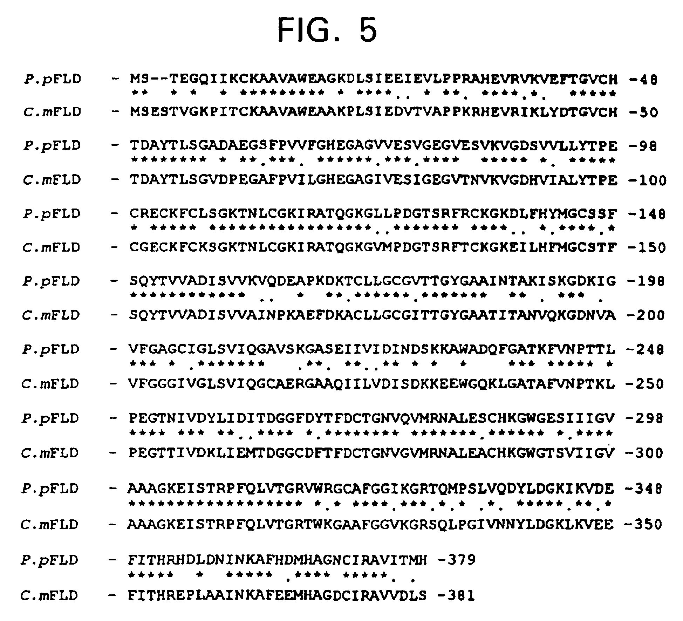

The GenBank/NCBI database was searched for other proteins with amino acid sequence similarity to the ORF product. The sequence of the putative FLD1 protein (Fldlp) showed the highest identity (71%) with that of glutathione-dependent formaldehyde dehydrogenase from the yeast *Candida maltosa* (Sasnauskas et al., 1992) (Fig. 5). *C. maltosa* is an *n*-alkane assimilating yeast and FLD is believed to be important in protecting the yeast from the toxic effects of formaldehyde (Sasnauskas et al., 1992). The close similarity of the predicted *C. maltosa* FLD product to that of the cloned ORF provides further support that this ORF encodes *P. pastoris* Fld1p. The *P. pastoris* Fld1p sequence also showed high identity with alcohol dehydrogenase III (ADHIII) proteins of higher eukaryotes (65%, human; 63%, horse; 64%, rat) and a lower but significant identity with other higher eukaryotic - ADHs (Holmquist and Vallee, 1991; Koivusalo et al., 1998; Rathnagiri et al., 1998). Finally, the Fld1p sequence showed little similarity with the predicted amino acid sequences of *S. cerevisiae* ADHs. The closest, at 19% identity, was *S. cerevisiae* ADHI (Jornvall et al., 1987).

### EXAMPLE 3

### Isolation and characterization of the Hansenula Polymorpha FLD gene

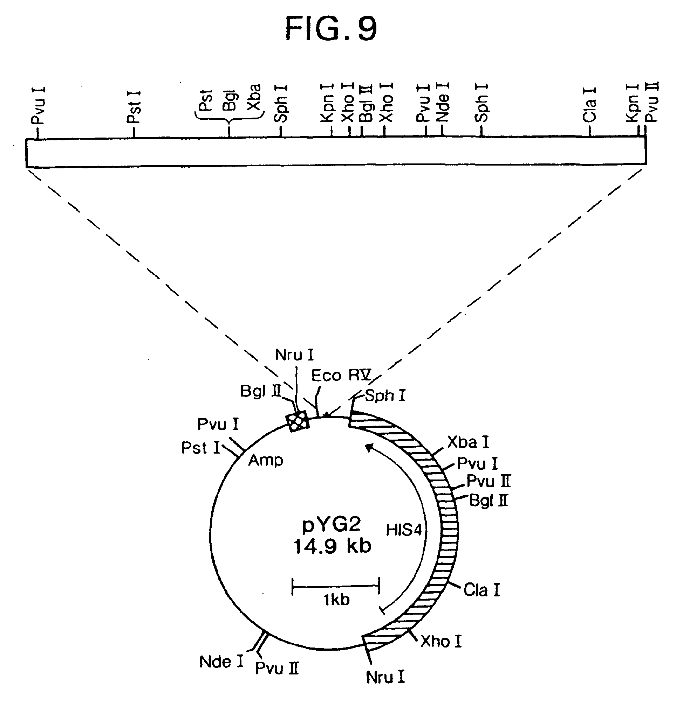

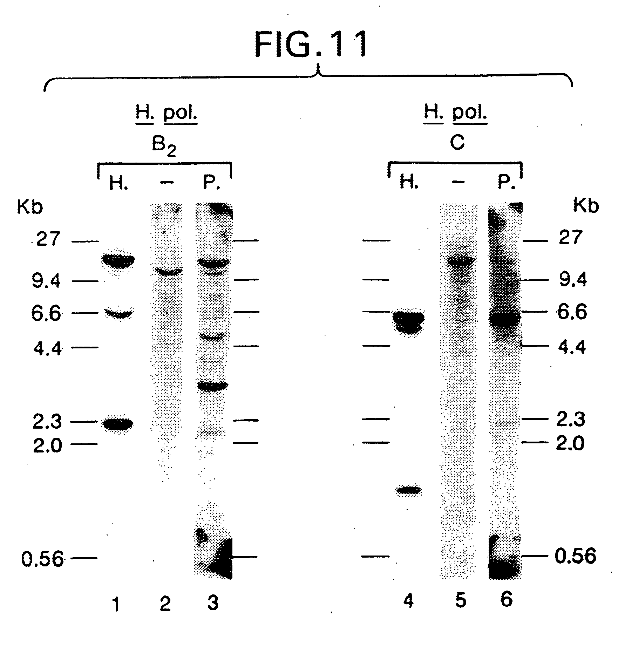

The putative *H*. *polymorpha FLD1* gene was isolated using the same functional complementation strategy described above for the *P*. *pastoris* gene. An *H*. *polymorpha* genomic DNA library was constructed in *P*. *pastoris* vector pYM8 in the same manner as the *P*. *pastoris* library (Liu et al. 1995). Briefly, *H*. *polymorpha* genomic DNA was partially digested with *Sau3A* and size selected for fragments of 5-20 kb. These fragments were ligated into the *BamHI* site of pYM8. The library was composed of approximately 100,000 independent *E. coli* transformants with greater than 90% containing an insert. The average size of insert DNA was approximately 10 kb. Assuming that the size of the *H. polymorpha* genome is 10,000 kb, the library contained approximately 100 genome equivalents of *H. polymorpha* genomic DNA. Plasmids were recovered and analyzed for ones that were capable of simultaneously retransforming MS105 (*f1d1-1 his4* to both His⁺ and Mut⁺ phenotypes. One plasmid, pYG2, (Figure 9)that met these criteria was selected for use in these studies. This plasmid contained an *H. polymorpha* DNA insert of 7.2 kb and the Mut complementing activity was found to reside within a 2.4-kb *Sph*I fragment (Figure 10). Southern blot studies indicated that vectors pYG1 and pYG2 contained homologous *FLD* genes. An example of such a blot is shown in Figures 11. In this experiment, genomic DNA from *H. polymorpha* was digested with either BglII (B₂) (lanes 1-3) or ClaI (C) (lanes 4-6) and hybridized with the following labeled probes: pYG2 (lanes 1 and 4), pYM8 (lanes 2 and 5), or pYG1 (lanes 3 and 6). The pYG2 probe containing the putative *H. polymorpha FLD1* gene produced major bands of ~15 kb and ~7 kb when hybridized at high stringency to BglII and ClaI digested *H. polymorpha* genomic DNAs, respectively. Hybridization of pYG1 containing the *P*. *pastoris FLD1* gene at low stringency (30% formamide, 37°C hybridization, lx SSC, room temperature washing) produced major bands of hybridization of the same size. These bands were not due to hybridization of vector sequences from pYM8, since the pYM8 probe showed no major bands of hybridization with *H*. *polymorpha* genomic DNA under the same low stringency conditions.

### EXAMPLE 4

### Comparison of the thermal stability of Fld1p from P. pastoris and H. polymorpha

Further evidence that the cloned *P*. *pastoris* gene actually encodes an FLD was obtained by comparing the thermal stability of its product to FLD from *H. polymorpha. H. polymorpha* is a related methylotrophic yeast that has a significantly higher optimal growth temperature than *P. pastoris* (42°C versus 30°C). FLD from *H. polymorpha* would therefore be expected to display a significantly higher thermal stability than *P. pastoris* FLD. A comparison of the thermal stability properties of the putative FLDs from the two yeasts provides strong support for the identity of the gene product. In this experiment, the putative *P*. *pastoris* and *H. polymorpha FLD1* genes were expressed in methanol-grown cells of the *P. pastoris fld1-1 his4* strain MS105, and the thermal stability of FLD activity in each was assessed by incubating extracts prepared from the strains at 60°C for selected periods of time and determining the rate of loss of FLD activity. If the genes actually encode Fld1p, the FLD inactivation rate for *H. polymorpha* Fld1p expressed in *P. pastoris* should be similar to that of wild-type *H. polymorpha* Fldlp, and the inactivation rate for the *P. pastoris* gene product should be similar to that of wild-type *P*. *pastoris* Fldlp.

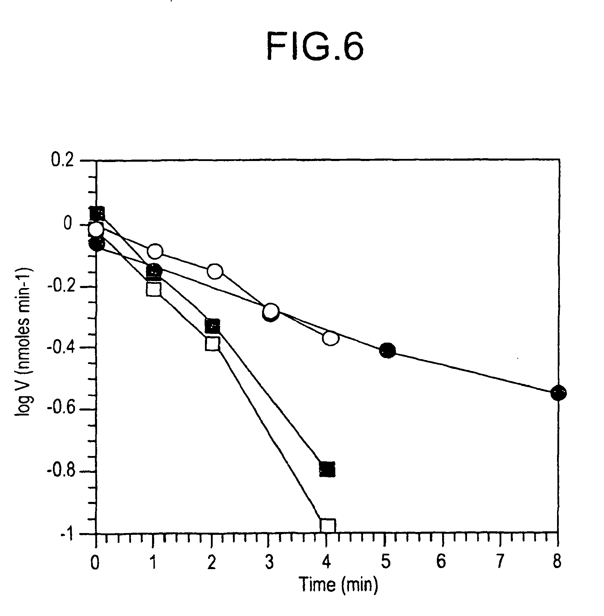

To perform this comparison, it was first necessary to establish that the thermal stability of the *P. pastoris* and *H. polymorpha* FLDs were significantly different and to clone the putative *H. polymorpha FLD1* gene. Thermal stabilities were determined by preparing cell-free extracts from methanol-grown cultures of wild-type *P. pastoris* and *H. polymorpha* and incubating them at 60°C. At selected times during incubation, samples of extract were removed and assayed for FLD activity. As shown in Fig. 6, *H. polymorpha* FLD activity was significantly more heat stable than *P*. *pastoris* activity.

Thermal stability of FLD expressed from *H*. *polymorpha* vector pYG2 was then compared to that of FLD from the *P. pastoris* vector pYG1. As shown in Fig. 6, FLD in MS105(pYG2) had a thermal inactivation rate similar to that of wild-type *H. polymorpha*, while MS105(pYG1) had a rate similar to that of *P. pastoris*. These results, taken with results demonstrating the specific absence of FLD activity in *P. pastoris* strain GS241 (and MS105) and the close similarity of the primary amino acid sequences of the cloned *P. pastoris* ORF and *C. maltosa* FLD, indicated that the cloned ORF encoded *P. pastoris* Fld1p.

### EXAMPLE 5

### Analysis of P_{FLD1} and comparison to P_{AOX1}

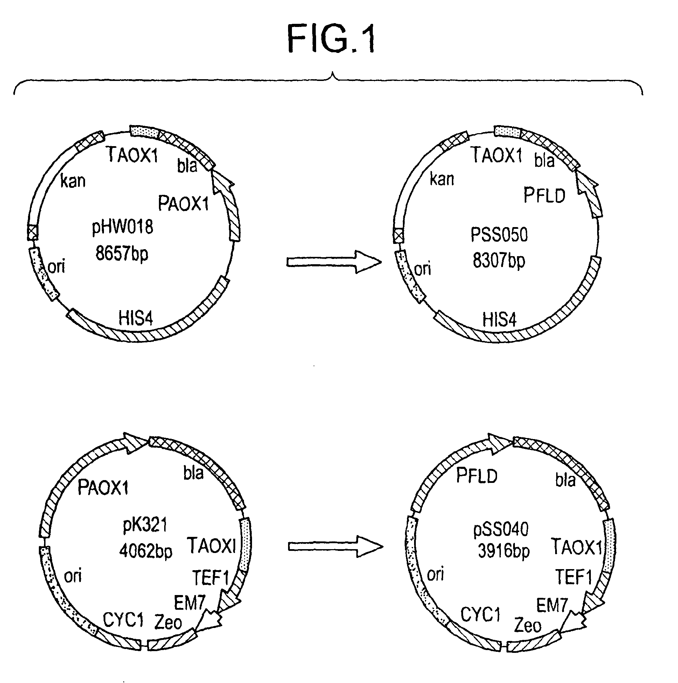

To examine gene expression under the transcriptional control of *P*_{*FLD1*}, two vectors were constructed (Fig. 1). Both vectors contained identical expression cassettes composed of a 0.6-kb *Mun*I*-Bam*HI fragment with sequences originating from just 5 ' of the methionine initiator ATG codon of *FLD1* fused to the bacterial *bla* gene encoding β-lactamase (β-lac), followed by a fragment containing the *AOX1* transcriptional terminator. The MunI is artificial and was installed by PCR using an oligonucleotide that contained the MunI site along with sequences from just 5' of the methionine initiator ATG of *FLD1*. A restriction site at this location was needed to aid in inserting the promoter 5' of the β-lactamase reporter gene. A MunI site was chosen because the DNA termini generated with MunI are compatible with EcoRI and there was an EcoRI site already present just 5' of the β-lactamase reporter in the test vectors. An EcoRI site could not be placed at the 3' end of the *FLD1* gene because the *FLD1* promoter region has a natural EcoRI site.

One vector, pSS040, contained a unique NsiI restriction site within the *P*_{*FLD1*} fragment. When cut at this site and transformed into *P*. *pastoris*, the vector efficiently integrated at the *P*_{*FLD1*} locus. The result of this integration event was a *P*_{*FLD1*}*-bla* expression cassette that also included native *FLD1* sequences upstream of the *P*_{*FLD1*} fragment (*WT-P*_{*FLD1*}*-bla)*. Assuming that all sequences required for transcriptional control of *FLD1* are located 5 ' of the *FLD1* ORF, regulation of *bla* and *FLD1* expression in this strain should be nearly identical. As shown in Table 2, this appeared to be true in that the relative levels of β-lac and FLD activity in the strain were similar in cells grown in four expression test media. These four media contained as carbon and nitrogen sources, respectively: (1) glucose and ammonium sulfate (G/NH₄⁺), (2) glucose and methylamine (G/MA), (3) methanol and ammonium sulfate (M/NH₄⁺), and (4) methanol and methylamine (M/MA). As expected, β-lac and FLD activities were highly repressed in cells grown on G/NH₄⁺ medium. Cells grown on either G/MA or M/NH₄⁺ media contained at least ten-fold more β-lac and FLD with the highest level of both enzymes observed in cells grown in M/MA medium.

The second vector, pSS050, contained the *P*. *pastoris HIS4* gene as the selectable marker. When cut at a unique *Sal*I site within *HIS4* and transformed in *P. pastoris*, this vector efficiently integrated at the *P. pastoris HIS4* locus. The result of this integration event was a *P*_{*FLD1*}*-b1a* expression cassette with sequences from pBR322 just 5' of the 0.6-kb *P*_{*FLD1*} fragment (*pB-P*_{*FLD1-*}*b1a*). Comparison of β-lac activity levels in this strain with those observed in the *WT-P*_{*FLD1*}*-b1a* strain allowed evaluation of whether the 0.6-kb fragment contained all upstream regulatory sequences required for normal regulation. Table 2 shows that β-lac activity levels in the *pB-P*_{*FLD1*}*-bla* strain were approximately two-fold higher than those observed in the *WT-P*_{*FLD1*}*-bla* strain when grown in each of the four expression test media. These results indicated that most sequences required for normal regulation were present within the *P*_{*FZD1*} fragment but that sequences that constitutively repress *P*_{*FLD1*} by a factor of about two-fold existed somewhere 5 ' of the *P*_{*FLD1*} fragment and were missing from the 0.6-kb fragment.

Finally, levels of β-lac activity produced under control of *P*_{*FLD1*} were compared with those of a strain in which *bla* expression was under the transcriptional control of *P*_{*AOX1*} (Waterham et al., 1997). As previously reported, *P*_{*AOX1*} expression is strongly repressed in the glucose-containing media and is highly and specifically induced in methanol-containing media (Tschopp et al., 1987; Waterham et al., 1997) (Table 2). Comparable levels of β-lac were present in cells of the *WT-P*_{*FLD1*}*-bla* strain grown in either M/NH₄⁺ or M/MA media, whereas cells of the *pB-P*_{*FLD1*}*-bla* strain contained levels of β-lac that were significantly higher than those in the *P*_{*AOX1*}*-bla* strain. Especially noteworthy were the levels of β-lac in the *pB-P*_{*FLD1*}*-bla* strain on M/NH₄⁺ and M/MA media which were consistently about twice those observed in the *P*_{*AOX1-*}*bla* strain on the same media.

### EXAMPLE 6

### The FLD1 gene confers resistance to formaldehyde

The *P. pastoris FLD1* gene was incorporated into pPICZ vectors containing *Zeo*^{*R*} (Invitrogen, Carlsbad, CA). Two such pPICZ-FLD1 vectors were constructed. In one, the whole *FLD1* gene including the *FLD1* promoter, structural gene and transcriptional terminator were inserted (pP_{FLD1}-FLD1). In the other, the *FLD1* structural gene (and transcriptional terminator) was placed under the control of the *P. pastoris* glyceraldehyde-3-phosphate dehydrogenase gene (GAP) promoter (pP_{GAP}-FLD1). These two plasmids were linearized within their respective promoter fragments (P _{FLD1}, P_{GAP}) and transformed by electroporation into wild-type and MS105 (*fld1-1 his4*) *P. pastoris* strains by selection for resistance to Zeocin at 100 µg/ml and 1 mg/ml. The lower Zeocin concentration selects for *P. pastoris* transformants that have one integrated copy of a *Zeo*^{*R*} vector while the high Zeocin concentration selects for transformants that have multiple integrated *Zeo*^{*R*} vector copies. Selected transformants of each type were streaked onto a YPD medium plate containing either Zeocin at 100 mg/ml or 1 mg/ml and onto sets of YPD plates containing formaldehyde at concentrations ranging from 0 to 30 mM. As a control, wild-type and GS241 strains transformed with a pPICZ vector alone (i.e., without an *FLD1* gene) were also streaked onto the plates.

It was observed that MS105 strains transformed with pPICZ alone were resistant to 1 mM formaldehyde. MS105-derived strains containing a single copy of pP_{FLD1}-FLD1 and pP_{GAP}-FLD1 were resistant to 10 mM and 5 mM formaldehyde, respectively, whereas MS105-derived strains containing multiple copies of pP_{FLD1} -FLD1 and pP_{GAP}-FLD1 were resistant to 30 mM and 10 mM formaldehyde, respectively. Thus, either the pP_{FLD1}-FLD1 and pP_{GAP}-FLD1 vectors conferred increased resistance to formaldehyde. In addition, an additive effect was evident in that increased numbers of copies of each vector resulted in an increased level of resistance to formaldehyde over that conferred by one copy of each vector.

Wild-type *P. pastoris* strains transformed with pPICZ alone were resistant to 5 mM formaldehyde. Because wild-type strains contain one native copy of the *FLD1* gene, the concentration of formaldehyde to which this strain was resistant was significantly higher as expected. Wild-type-derived strains containing a single copy of pP_{PLD1}-FLD1 and pP_{GAP}-FLD1 were resistant to 10 and 5 mM formaldehyde, respectively, whereas wild-type-derived strains containing multiple copies of pP_{FLD1}-FLD1 and pP_{GAP}-FLD1 were resistant to 30 mM formaldehyde, respectively. Thus, the pP_{FLD1}-FLD1 but not the pP_{GAP}-FLD1 vectors conferred increased resistance to formaldehyde. An additive effect was also evident with increased numbers of copies of each vector conferring an increased level of resistance to formaldehyde over one copy of each vector.

These transformation experiments were repeated with the pP_{FLD1}-FLD1 and PP_{GAP}-FLD1 vectors and wild-type and MS105 *P. pastoris* strains only selecting directly for resistance to formaldehyde (along with selection for Zeocin resistance as a control). With strain MS105, 2 mM formaldehyde was optimal for selection of transformants. This concentration of formaldehyde produced approximately the same number of transformants as observed with the 100 µg/ml Zeocin selection control. For wild-type *P. pastoris*, 7 mM formaldehyde was optimal for selection of transformants with the pP_{FLD1}-FLD1 vector. This concentration produced approximately the same number of transformants as observed with the 100 µg/ml Zeocin selection control. Transformation was not observed with the pP_{GAP}-FLD1 vector.

Based on these positive results, a *P*. *pastoris* expression vector was constructed. The vector contains a heterologous gene expression cassette composed of DNA fragments containing the *AOX1* promoter and transcriptional terminator separated by a multiple cloning site (MCS) into which heterologous genes can be inserted. The expression cassette is followed by a DNA segment containing the *P*_{*GAP*}*-FLD1* gene construct, and this segment is followed by a DNA fragment that is derived from sequences 3' of the *AOX1* gene. This set of DNA fragments is inserted into the bacterial plasmid pBluesceipt (Stratagene, San Diego, CA) so that the vector can be propagated in *E. coli.*

After insertion of the heterologous gene at the MCS, the resulting vector is cut with the restriction enzyme NotI to release from the bacterial plasmid a DNA fragment capable of transforming *P*. *pastoris.* The fragment is transformed into either wild-type or MS105 (*f1d1-1*) strains of *P. pastoris* by electroporation and transformants are selected on YPD medium plates containing either 7 mM formaldehyde for wild type strains or 2 mM for MS105 *fld1* strains. The vector fragment will insert itself into the *P*. *pastoris* genome in one of two ways. The first is by a gene replacement event replacing the *AOX1* gene. In addition to increased resistance to formaldehyde, such gene replacement transformants can be easily identified phenotypically because of their very slow growth rate on methanol due to the absence of the *AOX1* gene.

Another way the vector will insert itself into the *P*. *pastoris* genome involves the circularization of the transforming fragment at some point before integration. After circularization, the transforming DNA can integrate by a single cross-over event at any of the *P. pastoris* genomic loci represented in the vector. These genomic regions include the *FLD1, AOX1* promoter and *AOX1* 3' flanking loci. Integration at any of these sites produces no change in strain phenotype other than increased resistance to formaldehyde. It is important to note that integration of this fragment in any manner does not result in the incorporation of an antibiotic resistance gene or any other gene foreign to *P*. *pastoris* with the exception of the heterologous gene whose protein product is desired.

### References

Bradford, M. (1976) A rapid and sensitive method for the quantitation of microgram quantities of protein utilizing the principle of protein-dye binding. Anal. Biochem. 72, 248-254.
Cregg, J.M. Expression in the methylotrophic yeast *Pichia pastoris.* (1998) In: J. Fernandez and J. Hoeffler (Eds.), Nature: The Palette for the Art of Expression, Chapter 10. Academic Press, San Diego, in press.
Cregg, J.M., Barringer, K.J., Hessler, A.Y. and Madden, K.R. (1985) Pichia *pastoris* as a host system for transformations. Mol. Cell. Biol. 5, 3376.
Cregg, J.M. and Russell, K.A. Transformation. (1998) In: D.R. Higgins and J.M. Cregg (Eds.) Methods in Molecular Biology: Pichia Protocols, Chapter 3. Humana Press, Totowa, NJ, pp. 27-39.
Domdy, H., Apostol, B., Lin, R.J., Newman. A., Brody, E. and Abelson, J. (1984) Lariat structures are *in vivo* intermediates in yeast pre-mRNA splicing. Cell 39, 611-621.
Frohman, M.A., Dush, M.K. and Martin, G.R. (1988) Rapid production of full-length cDNAs from rare transcripts: amplification using a single gene-specific oligonucleotide primer. Proc. Natl. Acad. Sci. USA. 85, 8998-9002.
Higgins, D.R. and Cregg, J.M. (1998) Methods in Molecular Biology: Pichia Protocols. Humana Press, Totowa, New Jersey, pp. 1-15.
Holmquist, B. and Vallee, B.L. (1991) Human liver class III alcohol and glutathione dependent formaldehyde dehydrogenase are the same enzyme. Biochem. Biophys. Res. Commun. 178, 1371-1377.
Jornvall, H., Persson, B. and Jeffery, J. (1987) Characteristics of alcohol/polyol dehydrogenases: the zinc-containing long-chain alcohol dehydrogenases. Eur. J. Biochem. 167, 195-201.
Koivusalo, M., Barmann, M. and Uotila, L. (1989) Evidence for the identity of glutathione-dependent formaldehyde dehydrogenase and class III alcohol dehydrogenase. FEBS Lett. 257, 105-109.
Kramer, M.F. and Coen, D.M. The polymerase chain reaction. (1995) In: F.M. Ausubel, R. Brent, R.E. Kingston, D.D. Moore, J.G. Seidman, J.A. Smith and K. Struhl (Eds.) Current Protocols in Molecular Biology, Vol. 2, Chapter 15. John Wiley and Sons, New York, pp. 15.1.1-15.1.9.
Liu, H., Tan, X, Russell, K.A., Veenhuis, M. and Cregg, J.M. (1995) *PER3*, a gene required for peroxisome biogenesis in *Pichia pastoris,* encodes a peroxisomal membrane protein involved in protein import. J. Biol. Chem. 270, 10940-10951.
Lück, H. Catalase. (1963) In: H.U. Bergmeyer (Ed.), Methods of Enzymatic Analysis, Academic Press, San Diego, pp. 885-894.
Rathnagiri, P., Krug, J.F., Kozak, C., Moretti, T., O'Brien, S.J., Seuanez, H.N. and Goldman, D. (1989) Cloning and comparative mapping of human class III alcohol dehydrogenase cDNA. Biochem. Biophys. Res. Commun. 164, 453-460.
Sambrook, J., Fritsch, E.F. and Maniatis, T. (1989) Molecular Cloning: A Laboratory Manual, 2nd Edn. Cold Spring Harbor Laboratory Press, Cold Spring Harbor, NY. Sasnauskas, K., Jomantiene, R., Januska, A., Lebediene, E., Lebedys, J. and Janulaitis, A. (1992) Cloning and sequencing analysis of a *Candida maltosa* gene which confers resistance to formaldehyde in *Saccharomyces cerevisiae*. Gene 122, 207-211.
Schmitt, M., Brown, T.A. and Trumpower, B.L. (1990) A rapid and simple method for preparation of RNA from *Saccharomyces cerevisiae.* Nucleic Acids Res. 18, 3091-3092.
Schutte, H., Flossdorf, J., Sahm, H. and Kula, M.R. (1976) Purification and properties of formaldehyde dehydrogenase and formate dehydrogenase from *Candida boidinii*. Eur. J. Biochem. 62, 151-160.
Sibirny, A.A., Ubiyvovk, V.M., Gonchar, M.V., Titorenko, V.I., Voronovsky, A.Y., Kapultsevich, Y.G. and Bliznik, K.M. (1990) Reactions of direct formaldehyde oxidation to CO₂ are non-essential for energy supply of yeast methylotrophic growth. Arch. Microbiol 154, 566-575.
Sreekrishna, K. Strategies for optimizing protein expression and secretion in the methylotrophic yeast *Pichia pastoris*. (1993) In: R.H. Baltz, G.D. Hegeman, and P.L. Skatrud (Eds.), Industrial Microorganisms: Basic and Applied Molecular Genetics. American Society for Microbiology, Washington, DC, pp. 119-126.
Stewart, P., Kersten, P., Wymelenberg, A.V., Gaskell, J. and Cullen, D. (1992) Lignin peroxidase gene family of Phanerochaete *chrysosporium*: complex regulation by carbon and nitrogen limitation and identification of a second dimorphic chromosome. J. Bacteriol. 174, 5036-5042.
Tschopp, J.F., Brust, P.F., Cregg, J.M., Stillman, C.A. and Gingeras, T.R. (1987) Expression of the *lacZ* gene from two methanol-regulated promoters in *Pichia pastoris*. Nucleic Acids Res. 15, 3859-3876.
van Dijken, J.P., Harder, W., Beardsmore, A.J. and Quayle, J.R. (1978) Dihydroxyacetone: an intermediate in the assimilation of methanol by yeasts? FEMS Microbiol. Lett. 4, 97-102.
van Dijken, J.P. (1976) Oxidation of methanol by *Hansenula polymorpha:* purification and kinetic properties of methanol oxidase. Ph.D. Thesis, University of Groningen, pp. 30-43.
van der Klei, I.J., Bystrykh, L.V. and Harder, W. (1990) Alcohol oxidase from *Hansenula polymorpha* CBS 4732. Methods Enzymol. 188, 420-422.
Veenhuis, M., van Dijken, J.P. and Harder, W. (1983) The significance of peroxisomes in the metabolism of one-carbon compounds in yeasts. Adv. Microb. Physiol. 24, 1-82.
Waites, M.J. and Quayle, J.R. (1981) The interrelation between transketolase and dihydroxyacetone synthase activities in the methylotrophic yeast *Candida boidinii.* J. Gen. Microbiol. 124, 309-316.
Waterham, H.R., Keizer-Gunnink, I., Goodman, J.M., Harder, W. and veenhuis, M. (1992) Development of multipurpose peroxisomes in *Candida boidinii* grown in oleic acid-methanol limited continuous cultures. J. Bacteriol 174, 4057-4063.
Waterham, H.R., Digan, M.E., Koutz, P.J., Lair, S.V. and Cregg, J.M. (1997) Isolation of the Pichia *pastoris* glyceraldehyde-3-phosphate dehydrogenase gene and regulation and use of its promoter. Gene 186, 37-44.
Zwart, K., Veenhuis, M., van Dijken, J.P. and Harder, W. (1980) Development of amine oxidase-containing peroxisomes in yeast during growth on glucose in the presence of methylamine as the sole source of nitrogen. Arch. Microbiol. 126, 117-126.

**Table 1 Relative enzyme activity levels in methanol-utilization-defective mutants of P. pastoris.**

| Strain | % Activity^{a} | | | | | |
|---|---|---|---|---|---|---|
| | AOX | CAT | FLD | FDH | DAS | DAK |
| WT (methanol) | 100 | 100 | 100 | 100 | 100 | 100 |
| WT (glucose) | 0 | 2 | 1 | 0 | 3 | 53 |
| KM7121 (*aox1 aox2*) | 0 | 100 | 26 | 31 | ND^{b} | 88 |
| GS241 (*fld1*) | 20 | 178 | 0 | 46 | 58 | 64 |

| | | | | | | |
|---|---|---|---|---|---|---|
| ^{a} Activity for each enzyme is expressed as a percentage of that observed in.extracts prepared from methanol-grown cultures of wild-type *P. pastoris.* Abbreviations are: AOX, alcohol oxidase; CAT, catalase; FLD, formaldehyde dehydrogenase; FDH, formate dehydrogenase; DAS, dihydroxyacetone synthase; DAK, dihydroxyacetone kinase. | | | | | | |
| ^{b} Not determined. | | | | | | |

**Table 2 Comparison of β-lactamase activity in extracts of P. pastoris strains expressing bla under control of P_{FLD} and P_{AOX1}.**

| Strain | Source of:^{a} | | Enzyme activity^{b} | | | |
|---|---|---|---|---|---|---|
| | C | N | β-lactamase | | FLD | |
| *WT-P*_{*FLD1*}*-bla* (at *FLD1* locus) | G | NH₄⁺ | 14 | (4%) | 0.13 | (6%) |
| | G | MA | 168 | (48%) | 1.50 | (69%) |
| | M | NH₄⁺ | 310 | (88%) | 1.69 | (78%) |
| | M | MA | 352 | (100%) | 2.16 | (100%) |
| *pB-P*_{*FLD1*}*-bla* (at *HIS4* locus) | G | NH₄⁺ | 19 | (5%) | 0.11 | (5%) |
| | G | MA | 357 | (102%) | 0.82 | (38%) |
| | M | NH₄⁺ | 529 | (150%) | 1.48 | (69%) |
| | M | MA | 530 | (151%) | 1.75 | (81%) |
| *P*_{*AOX1*}*-bla* | G | NH₄⁺ | 0.3 | (0.1%) | 0.12 | (6%) |
| | G | MA | 0.5 | (0.1%) | 0.65 | (30%) |
| | M | NH₄⁺ | 241 | (68%) | 1.40 | (65%) |
| | M | MA | 254 | (72%) | 2.06 | (95%) |

| | | | | | | |
|---|---|---|---|---|---|---|
| ^{a} Each strain was grown in media containing either glucose (G) or methanol (M) as carbon source and ammonium sulfate (NH₄⁺) or methylamine (MA) as nitrogen source. | | | | | | |
| ^{b} β-lactamase activities are expressed as nmol/mg per min and, in parentheses, as a percentage of activity seen in the *WT-P*_{*FLD1*}*-bla* strain grown on methanol and methylamine. Activities represent the mean of three experiments using two independently transformed strains. | | | | | | |

## Claims

1. An isolated nucleic acid comprising a promoter that is inducible by methanol, methylamine, and a mixture of both methanol and methylamine in a methylotrophic yeast, wherein said isolated nucleic acid is derivable from Pichia pastoris.

2. An isolated nucleic acid according to claim 1 comprising the sequence set forth in SEQ ID NO: 3.

3. An isolated nucleic acid comprising a promoter according to claims 1 or 2, further comprising a formaldehyde dehydrogenase coding sequence (FLD) derivable from Pichia pastoris, wherein said coding sequence is sufficient to complement an fld- defect in a Pichia pastoris strain.

4. An isolated nucleic acid according to claim 3 comprising a sequence encoding the amino acid sequence set forth in SEQ ID NO:2.

5. An isolated nucleic acid according to claim 3 or 4 comprising the sequence set forth in SEQ ID No: 1 or SEQ ID No: 5, or a nucleotide sequence complementary to the nucleotide sequence set forth in SEQ ID No: 1 or SEQ ID No: 5.

6. An isolated nucleic acid comprising a formaldehyde dehydrogenase coding sequence (FLD) from a methylotrophic yeast, wherein said coding sequence comprises a sequence encoding the amino acid sequence set forth in SEQ ID NO:2.

7. An isolated nucleic acid comprising the sequence set forth in SEQ ID NO: 5

8. The isolated nucleic acid of any of claims 1 to 7 having a length of 2.7 kb.

9. An isolated polypeptide comprising the amino acid sequence set forth in SEQ ID NO:2.

10. A vector comprising the isolated nucleic acid of any one of claims 1 to 8.

11. A nucleic acid expression cassette which comprises the promoter of any one of claims 1 to 3, wherein said promoter is operably linked to a nucleic acid encoding a heterologous protein.

12. The expression cassette of claim 11 further comprising a 3' termination sequence.

13. The expression cassette of claim 11 or 12, wherein the 3' termination sequence is that of the AOX1 gene.

14. An expression vector which comprises an expression cassette according to any of claims 11 to 13.

15. A Pichia pastoris cell transformed with a nucleic acid according to any one of claims 10 to 14.

16. A method for directing expression of a heterologous coding sequence in Pichia pastoris which comprises:
a) introducing into a Pichia pastoris cell an isolated nucleic acid comprising a promoter according to claim 1 or 2 wherein said promoter is operably linked at its 3' end to the 5' end of a heterologous coding sequence, said heterologous coding sequence operably linked at its 3' end to the 5' end of a termination sequence which functions in Pichia pastoris;
b) growing said Pichia pastoris cell in a medium having a suitable carbon source such as glycerol or glucose, and having a suitable nitrogen source such as an ammonium salt or ammonium hydroxide, and after the carbon or nitrogen source is depleted;
c) inducing expression of said heterologous coding sequence by addition of methanol or methylamine or both methanol and methylamine.

17. A method for directing expression of a heterologous coding sequence in Pichia pastoris which comprises:
a) introducing into a Pichia pastoris cell an isolated nucleic acid comprising a promoter according to claim 1 or 2 wherein said promoter is operably linked at its 3' end to the 5' end of a heterologous coding sequence, said heterologous coding sequence operably linked at its 3' end to the 5' end of a termination sequence which functions in Pichia pastoris;
b) growing said Pichia pastoris cell in a medium having a suitable carbon source such as glycerol or glucose, and having a suitable nitrogen source such as an ammonium salt or ammonium hydroxide, and after the carbon or nitrogen source is depleted;
c) inducing expression of said heterologous coding sequence by addition of formaldehyde, formate, or a methylated amine.

18. The method of claim 17 wherein the methylated amine is choline.

## Patentansprüche

1. Isolierte Nukleinsäure, umfassend einen Promotor, der durch Methanol, Methylamin und ein Gemisch aus sowohl Methanol als auch Methylamin in einer methylotrophen Hefe induzierbar ist, worin die isolierte Nukleinsäure aus Pichia pastoris ableitbar ist.

2. Isolierte Nukleinsäure gemäß Anspruch 1, umfassend die in SEQ ID NO: 3 angeführte Sequenz.

3. Isolierte Nukleinsäure, umfassend einen Promotor gemäß Ansprüchen 1 oder 2, ferner umfassend eine für Formaldehyddehydrogenase kodierende Sequenz (FLD), welche aus Pichia pastoris ableitbar ist, worin diese kodierende Sequenz ausreichend ist, um einen fld-Defekt in einem Pichia-pastoris-Stamm zu komplementieren.

4. Isolierte Nukleinsäure gemäß Anspruch 3, umfassend eine Sequenz, welche für die in SEQ ID NO: 2 angeführte Aminosäuresequenz kodiert.

5. Isolierte Nukleinsäure gemäß Ansprüchen 3 oder 4, umfassend die in SEQ ID NO: 1 oder SEQ ID NO: 5 angeführte Sequenz, oder eine Nukleotidsequenz, die zu der in SEQ ID NO: 1 oder SEQ ID NO: 5 angeführten Nukleotidsequenz komplementär ist.

6. Isolierte Nukleinsäure, umfassend eine für eine Formaldehyddehydrogenase kodierende Sequenz (FLD), aus einer methylotrophen Hefe, worin diese kodierende Sequenz eine Sequenz, die für die in SEQ ID NO: 2 angeführte Aminosäuresequenz kodiert, umfasst.

7. Isolierte Nukleinsäure, umfassend die in SEQ ID NO: 5 angeführte Sequenz.

8. Isolierte Nukleinsäure gemäß einem der Ansprüche 1-7, mit einer Länge von 2,7 kb.

9. Isoliertes Polypeptid, umfassend die in SEQ ID NO: 2 angeführte Aminosäuresequenz.

10. Vektor, umfassend die isolierte Nukleinsäure gemäß einem der Ansprüche 1-8.

11. Nukleinsäureexpressionskassette, umfassend den Promotor gemäß einem der Ansprüche 1-3, worin der Promotor funktionell mit einer Nukleinsäure, welche für ein heterologes Protein kodiert, verknüpft ist.

12. Expressionskassette gemäß Anspruch 11, ferner umfassend eine 3'-Terminationssequenz.

13. Expressionskassette gemäß Ansprüchen 11 oder 12, worin die 3'-Terminationssequenz jene des AOX1-Gens ist.

14. Expressionsvektor, umfassend eine Expressionskassette gemäß einem der Ansprüche 11-13.

15. Pichia-pastoris-Zelle, welche mit einer Nukleinsäure gemäß einem der Ansprüche 10-14 transformiert ist.

16. Verfahren zur Regelung der Expression einer heterologen kodierenden Sequenz in Pichia pastoris, umfassend:
a) Das Einführen in eine Pichia-pastoris-Zelle einer isolierten Nukleinsäure, umfassend einen Promotor gemäß Ansprüchen 1 oder 2, worin der Promotor an seinem 3'-Ende funktionell mit dem 5'-Ende einer heterologen kodierenden Sequenz verknüpft ist, wobei die heterologe kodierende Sequenz an ihrem 3'-Ende mit dem 5'-Ende einer Terminationssequenz, die in Pichia pastoris funktioniert, funktionell verknüpft ist;
b) das Heranwachsenlassen der Pichia-pastoris-Zelle in einem Medium mit einer geeigneten Kohlenstoffquelle, wie beispielsweise Glycerin oder Glukose, und mit einer geeigneten Stickstoffquelle, wie beispielsweise einem Ammoniumsalz oder Ammoniumhydroxid, und nachdem die Kohlenstoff- oder Stickstoffquelle aufgebraucht ist;
c) das Induzieren der Expression der heterologen kodierenden Sequenz durch Hinzugabe von Methanol oder Methylamin oder sowohl Methanol als auch Methylamin.

17. Verfahren zur Regelung der Expression einer heterologen kodierenden Sequenz in Pichia pastoris, umfassend:
a) Das Einführen in eine Pichia-pastoris-Zelle einer isolierten Nukleinsäure, umfassend einen Promotor gemäß Ansprüchen 1 oder 2, worin der Promotor an seinem 3'-Ende funktionell mit dem 5'-Ende einer heterologen kodierenden Sequenz verknüpft ist, wobei die heterologe kodierende Sequenz an ihrem 3'-Ende mit dem 5'-Ende einer Terminationssequenz, die in Pichia pastoris funktioniert, funktionell verknüpft ist;
b) das Heranwachsenlassen der Pichia-pastoris-Zelle in einem Medium mit einer geeigneten Kohlenstoffquelle, wie beispielsweise Glycerin oder Glukose, und mit einer geeigneten Stickstoffquelle, wie beispielsweise einem Ammoniumsalz oder einem Ammoniumhydroxid, und nachdem die Kohlenstoff- oder Stickstoffquelle aufgebraucht ist;
c) das Induzieren der Expression der heterologen kodierenden Sequenz durch die Hinzugabe von Formaldehyd, Format oder eines methylierten Amins.

18. Verfahren gemäß Anspruch 17, worin das methylierte Amin Cholin ist.

## Revendications

1. Acide nucléique isolé comprenant un promoteur qui est inductible par le méthanol, la méthylamine et un mélange de méthanol et de méthylamine dans une levure méthylotrophique, ledit acide nucléique isolé pouvant être dérivé de Pichia pastoris.

2. Acide nucléique isolé selon la revendication 1 comprenant la séquence représentée par SEQ ID N° : 3.

3. Acide nucléique isolé comprenant un promoteur selon la revendication 1 ou 2, comprenant en outre une séquence codante de formaldéhyde déshydrogénase (FLD) pouvant être dérivée de Pichia pastoris, dans lequel ladite séquence codante est suffisante pour complémenter un défaut de fld dans une souche de Pichia pastoris.

4. Acide nucléique isolé selon la revendication 3 comprenant une séquence codant pour la séquence d'acides aminés représentée par SEQ ID N° : 2.

5. Acide nucléique isolé selon la revendication 3 ou 4 comprenant la séquence représentée par SEQ ID N° : 1 ou SEQ ID N° : 5, ou une séquence nucléotidique complémentaire de la séquence nucléotidique représentée par SEQ ID N° : 1 ou SEQ ID N° : 5.

6. Acide nucléique isolé comprenant une séquence codante de formaldéhyde déshydrogénase (FLD) provenant d'une levure méthylotrophique, dans lequel ladite séquence codante comprend une séquence codant pour la séquence d'acides aminés représentée par SEQ ID N° : 2.

7. Acide nucléique isolé comprenant la séquence représentée par SEQ ID N° : 5.

8. Acide nucléique isolé selon l'une quelconque des revendications 1 à 7 ayant une longueur de 2,7 kb.

9. Polypeptide isolé comprenant la séquence d'acides aminés représentée par SEQ ID N° : 2.

10. Vecteur comprenant l'acide nucléique isolé selon l'une quelconque des revendications 1 à 8.

11. Cassette d'expression d'acide nucléique qui comprend le promoteur selon l'une quelconque des revendications 1 à 3, dans laquelle ledit promoteur est fonctionnellement lié à un acide nucléique codant pour une protéine hétérologue.

12. Cassette d'expression selon la revendication 11 comprenant en outre une séquence de terminaison 3'.

13. Cassette d'expression selon la revendication 11 ou 12, dans laquelle la séquence de terminaison 3' est celle du gène AOX1.

14. Vecteur d'expression qui comprend une cassette d'expression selon l'une quelconque des revendications 11 à 13.

15. Cellule de Pichia pastoris transformée avec un acide nucléique selon l'une quelconque des revendications 10 à 14.

16. Procédé visant à diriger l'expression d'une séquence codante hétérologue dans Pichia pastoris qui comprend :
a) l'introduction dans une cellule de Pichia pastoris d'un acide nucléique isolé comprenant un promoteur selon la revendication 1 ou 2 dans lequel ledit promoteur est fonctionnellement lié à son extrémité 3' à l'extrémité 5' d'une séquence codante hétérologue, ladite séquence codante hétérologue étant fonctionnellement liée à son extrémité 3' à l'extrémité 5' d'une séquence de terminaison qui fonctionne dans Pichia pastoris ;
b) la croissance de ladite cellule de Pichia pastoris dans un milieu comportant une source de carbone appropriée telle que du glycérol ou du glucose, et comportant une source d'azote appropriée telle qu'un sel d'ammonium ou un hydroxyde d'ammonium, et une fois que la source de carbone ou d'azote est épuisée ;
c) l'induction de l'expression de ladite séquence codante hétérologue par addition de méthanol ou de méthylamine ou à la fois de méthanol et de méthylamine.

17. Procédé visant à diriger l'expression d'une séquence codante hétérologue dans Pichia pastoris qui comprend :
a) l'introduction dans une cellule de Pichia pastoris d'un acide nucléique isolé comprenant un promoteur selon la revendication 1 ou 2 dans lequel ledit promoteur est fonctionnellement lié à son extrémité 3' à l'extrémité 5' d'une séquence codante hétérologue, ladite séquence codante hétérologue étant fonctionnellement liée à son extrémité 3' à l'extrémité 5' d'une séquence de terminaison qui fonctionne dans Pichia pastoris ;
b) la croissance de ladite cellule de Pichia pastoris dans un milieu comportant une source de carbone appropriée telle que du glycérol ou du glucose, et comportant une source d'azote appropriée telle qu'un sel d'ammonium ou un hydroxyde d'ammonium, et une fois que la source de carbone ou d'azote est épuisée ;
c) l'induction de l'expression de ladite séquence codante hétérologue par addition de formaldéhyde, de formate ou d'une amine méthylée.

18. Procédé selon la revendication 17 dans lequel l'amine méthylée est la choline.
